# EUROPEAN PATENT SPECIFICATION

(11) **EP 1 666 020 B1**
(45) Date of publication and mention of the grant of the patent: **02.05.2012**
(21) Application number: 04771843.2
(22) Date of filing: 19.08.2004
(51) Int. Cl.: A61K 9/06, A61K 47/02, A61K 47/18, A61K 47/36, A61K 33/10

(54) **Compositions for the preparation of carbon dioxide gel for external use and carbon dioxide gels for external use**
Zusammensetzungen zur Herstellung von Kohlendioxid-Gels zur topischen Anwendung und Kohlendioxid-Gels zur topischen Anwendung
Compositions pour preparer un gel au dioxyde de carbone pour utilisation externe, et gels au dioxyde de carbone pour utilisation externe

(30) Priority: 19.08.2003 JP 2003295443
(43) Date of publication of application: 07.06.2006
(73) Proprietor: Neochemir, Inc., Kobe-shi, Hyogo 651-0087 (JP)
(72) Inventor: TANAKA, Masaya, Suma-ku, Kobe-shi, Hyogo 6540036 (JP); WEI, Chunhong, Kobe-shi, Hyogo 658-0032 (JP)
(74) Representative: Sajda, Wolf E.
(86) International application number: PCT/JP2004/011882
(87) International publication number: WO 2005/016290

(56) References cited:
- EP-A- 0 380 253
- EP-A- 1 043 023
- EP-A- 1 541 155
- WO-A1-02/080941
- JP-A- 2000 319 187
- JP-A- 2003 073 253
- US-A- 4 678 661
- US-A- 4 721 614

## Description

### Technical Field

The present invention relates to compositions for the preparation of carbon dioxide gel for external use which readily delivers aesthetic or medial effects through external use of carbon dioxide, and to carbon dioxide gels for external use prepared from the compositions.

### Background Art

It is apparent that transdermally or transmucosally absorbed carbon dioxide acts to promote blood circulation in dermal and subcutaneous tissues and in muscles so as to activate metabolism, thereby providing the aesthetic or medical effects from the fact that carbonate spring has been used for this purpose in various parts of the world, or is suggested in various study reports. As an example of the medical effects, Hiyoshi et al report that artificial carbonate spring is effective in treating intractable decubitus (Hiyoshi, Toshinori; Treatment of Decubitus Using Artificial Carbonate Spring Preparation, Comprehensive Rehabilitation 17(8): 605-609, 1989).

In an attempt to readily derive the aesthetic or medical effects from external use of carbon dioxide, a carbon dioxide agent for external use incorporating carbon dioxide and an external agent adapted to generate carbon dioxide that is to be prepared at use have been proposed. The former carbon dioxide agent for external use has problems that a technique for the manufacturing process thereof has yet to be established and that a storage container ensuring no or little loss of carbon dioxide is expensive. In contrast, the external agent adapted to generate carbon dioxide that is prepared at use does not involve these problems and is more useful. A kit for preparation of composition for use in transdermal/transmucosal absorption of carbon dioxide (Japanese Patent Application Laid-open No.2000-319187) and a composition for use in preparation of carbon dioxide agent for external use (WO 02/80941), for example, have been proposed as compositions providing for the preparation of such an agent for external use.

Japanese Patent Application Laid-open No.2000-319187 and WO 02/80941 disclose that transdermally or transmucosally absorbtion of carbon dioxide is effective against pruritus accompanying dermatomucosal diseases or disorders such as athlete's foot, insect bite, atopic dermatitis, nummular eczema, xeroderma, seborrheic eczema, urticaria, prurigo, housewives' eczema, acne vulgaris, impetigo, folliculitis, carbuncles, furuncles, phlegmon, pyoderma, psoriasis, ichthyosis, palmoplantar keratoderma, lichen, pityriasis, wounds, burns, rhagades, erosion and chilblains; dermatomucosal injuries such as decubitus, wounds, burns, angular stomatitis, stomatitis, skin ulcers; rhagades, erosion, chilblains and gangrene; graft failure of skin grafts, skin flaps and the like; dental diseases such as gingivitis, alveolar pyorrhea, denture ulcers, nigricans gingival and stomatitis; skin ulcers, cryesthesia and numbness caused by peripheral circulatory disorders such as thromboangitis obliterans, arteriolosclerosis obliterans, diabetic peripheral circulatory disorders and lower limb varicosis; musculoskeletal diseases such as chronic rheumatoid arthritis, cervico-omo-brachial syndrome, myalgia, arthralgia and lumbago; nervous system diseases such as neuralgia, polyneuritis and subacute myelo-optic neuropathy; keratoses such as proriasis, clavus, callus, ichthyosis, palmoplantar keratoderma, lichen and pityriasis; suppurative skin diseases such as acne vulgaris, impetigo, folliculitis, carbuncles, furuncles, phlegmon, pyoderma and suppurative eczema; suppression of regrowth of hair after depilation (waxing); cosmetic problems concerning facial skin and hair such as freckles, skin dryness, hyperpigmented skin, decline of skin fitness and luster and decline of hair luster; and local obesity.

It is generally known that carbon dioxide in gas phase is practically unabsorbable through skin or mucosa. That the carbonate spring provides the medical and aesthetic effects, such as to lower the blood pressure, to promote the healing of decubitus and to improve skin dryness, is because carbon dioxide dissolved in the water of carbonate spring is transdermally or transmucosally absorbed (Nagakura, Isao; "Carbon Dioxide Gas, A Wonderful Substance Sustaining Life", published from Asahi Shinbun Company). It is therefore necessary to dissolve carbon dioxide in a solvent such as water in order to obtain the above aesthetic or medical effects.

Because the kit disclosed in Japanese Patent Application Laid-open No.2000-319187 uses carbon dioxide in a bubble form the resultant composition does not achieve high transdermal or transmucosal absorbance when it is applied to skin or mucosa. Therefore, a long-term, continued use of the composition is required for deriving adequate aesthetic or medical effects from the composition. Specifically, daily use of the composition for a period of two weeks to two months is required to achieve a face slimming effect. The carbon dioxide in the bubble form is not utilized for the transdermal or transmucosal absorption purpose because carbon dioxide bubbles form useless air cells in the composition prepared using the above kit. In addition, the carbon dioxide bubbles also have a drawback of increasing the fluidity of the whole composition so that the composition may drop down from applied surface to soil clothes. Furthermore, the composition obtained from the above kit is a viscous material and hence, the removal of the composition after use requires face washing. It cannot be said that the above composition is easy to use.

On the other hand, the composition disclosed in the publication No.WO 02/80941 overcomes the most of the drawbacks of the kit disclosed in Japanese Patent Application Laid-open No.2000-319187. However, the removal of the composition after use requires face washing. Hence, the composition may not quite definitely be said to be convenient. Although the composition affords more rapid and higher aesthetic or medical effects than the aforementioned kit, further improvement of the composition is desired in terms of efficacy.

The intermediate publication EP1541155 describes a material of carbon dioxide external preparation comprising a polymeric three-dimensional network impregnated with viscous material having an acid and water. The reactant carbonate contacts with viscous phase and generates carbon dioxide in a non-bubble state.

In view of the foregoing, the present invention has an object to provide a composition for use in preparation of a carbon dioxide gel for external use which affords more rapid and higher aesthetic or medical effects, which does not drop down when applied and which is easy to remove after use, as well as to provide a carbon dioxide gel for external use prepared from the composition.

### Disclosure of the Invention

The present inventors have devoted themselves to an intensive study on carbon dioxide agents for external use providing the higher aesthetic or medical effects. In the study process, the inventors contemplated to gelate the carbon dioxide agent for external use by using cation released from carbonate as a carbon dioxide source material and to allow carbon dioxide in the non-bubble form to be transdermally or transmucosally absorbed for achieving the higher aesthetic or medical effects. While carrying on the experimental work, the inventors encountered the following problems. That is, carbonate reacts rapidly with acid to cause a rapid generation of carbon dioxide, whereas a reaction between the cation released from carbonate and a gelling agent is also rapid. Hence, the resultant carbon dioxide forms bubbles in the gel or leaks out to the atmosphere, and depending upon the materials used or mixing ratios thereof, the generation of carbon dioxide may end before the gelation is completed. Therefore, the inventors further made the experimental work aiming at overcoming the foregoing problems by reducing the generating rate and the dissolving rate of carbon dioxide and the reaction rate of the gelling agent so as to synchronize the generating rate of carbon dioxide with the gelating rate. Consequently, the inventors have accomplished the invention.

The first composition for use in preparation of carbon dioxide gel for external use according to the present invention is used for the preparation of a carbon dioxide gel for external use wherein the carbon dioxide is dissolved in a substantial non-bubble form, and comprises the following granular material (A) and viscous material (B) to be mixed with the granular material (A):
(A) a granular material comprising a weak acid a dispersant and a calcium ion trapping agent as essential components;
(B) a viscous material comprising calcium carbonate, a gelling agent to be gelated with calcium ions and water as essential components.

The term "gel", as used herein, means one which comprises macromolecules swelled in a solvent such as water to form a three-dimensional network in which a large amount of solvent molecules are unreleasably captured.
The term "substantial non-bubble form", as used herein, means a state where carbon dioxide bubbles are so small as to be hardly visible to the naked eye.
The term "granular material", as used herein, means a solid substance such as powder, fine particles, granulated powder and microcapsule, and mixtures thereof.
The term "calcium ion trapping agent", as used herein, means an agent which is able to combine with calcium ions and is able to control the gelation rate of the gelling agent which is gelated by calcium ions and the solidification rate of the gel.

According to the first composition for use in preparation of carbon dioxide gel for external use, the mixing of the above granular material (A) with the above viscous material (B) causes the weak acid in the granular material (A) to react with calcium carbonate in the viscous material (B) so as to generate carbon dioxide. In the meantime, the gelling agent, which is present in the viscous material (B) and gelated by the calcium ions (hereinafter, simply referred to as "gelling agent") is gelated by calcium ions released from the above calcium carbonate, and the resultant gel is solidified. In this process, the calcium ion trapping agent traps the calcium ions so that a rapid progress of the gelation is suppressed. Furthermore, the calcium ion trapping agent in the granular material (A) acts to retard contact between the weak acid and calcium carbonate during the mixing of the granular material (A) and the viscous material (B) and hence, the rapid generation of carbon dioxide is also obviated. This is effective to prevent the problems that the gel is solidified before carbon dioxide in the non-bubble form is dissolved, that the rapid generation of carbon dioxide detrimentally allows carbon dioxide bubbles to remain in the gel, or that the generated carbon dioxide leaks out into the atmosphere. Therefore, the carbon dioxide gel for external use can be obtained in which a large amount of carbon dioxide in the substantial non-bubble form is dissolved. Such a carbon dioxide gel for external use affords high transdermal or transmucosal absorption of carbon dioxide and hence, even higher aesthetic or medial effects can be achieved. What is more, the composition in the gel form is less prone to drop down on the skin or mucosa. In addition, the mixing ratios or the like of the ingredients can be adjusted such that the gel can be solidified after the lapse of a predetermined length of time from preparation and can be easily pealed off from the skin and mucosa.

In the first composition for use in preparation of carbon dioxide gel for external use, it is preferred that the calcium ion trapping agent of the above granular material (A) is at least one of disodium ethylenediamine tetraacetate and glycine. In this case, the calcium ion trapping agent more effectively traps the calcium ions so that the rapid gelation is more effectively suppressed. Accordingly, the even higher aesthethic or medical effects can be more effectively derived from the carbon dioxide gel for external use prepared from such a composition for use in preparation of carbon dioxide gel for external use.

In the first composition for use in preparation of carbon dioxide gel for external use, it is preferred that the weak acid in the granular material (A) is at least one of sodium dihydrogenphosphate and potassium dihydrogenphosphate; that the calcium ion trapping agent of the above granular material (A) is at least one of disodium ethylenediamine tetraacetate and glycine; and that the gelling agent of the viscous material (B), which is gelated by the calcium ions, is sodium alginate. In this case, it is advantageously easy to prepare the carbon dioxide gel for external use having carbon dioxide dissolved therein in the substantial non-bubble form. Furthermore, the end time of the generation/dissolution of carbon dioxide is substantially synchronized with the end time of the reaction of the gelling agent. Thus, the composition has an advantage of providing the carbon dioxide gel for external use which delivers even higher aesthetic or medical effects in a more effective manner. In addition, the above composition offers an easy preparation of the carbon dioxide gel for external use which is less prone to drop down on the skin/mucosa. The composition has another advantage that if the mixing ratios or the like of the ingredients are adjusted, a carbon dioxide gel for external use can be readily prepared which is solidified after the lapse of a predetermined length of time from preparation, thus providing for the easy peeling-off thereof.

In the first composition for use in preparation of carbon dioxide gel for external use, the granular material (A) further contains a dispersant. In this case, the contact between the weak acid and calcium carbonate is suppressed when the granular material (A) is dissolved in the viscous material (B). Hence, the rate of generation of carbon dioxide can be reduced even further. This permits the generated carbon dioxide in the substantial non-bubble form to be directly dissolved in the gel, without forming bubbles in the gel or leaking out into the atmosphere. In the meantime, calcium ions released from calcium carbonate are also reduced in release rate, so that the gelling agent can be gelated and solidified at proper rates. That is, when the carbon dioxide gel for external use is prepared using the granular material (A) and the viscous material (B), a gel which is slowly solidified and in which a required amount of carbon dioxide in the substantial non-bubble form is dissolved can be formed. Therefore, when the granular material (A) is mixed with the viscous material (B) and the resultant mixture is applied to the skin or mucosa, carbon dioxide in the substantial non-bubble form is transdermally or transmucosally absorbed to afford the high aesthetic or medical effects. In addition, the resultant carbon dioxide gel for external use is less prone to drop down on the skin or mucosa because of the gel nature. If the mixing ratios or the like of the ingredients are adjusted, the formed gel can be solidified after the lapse of a predetermined length of time from preparation, so as to be easily pealed off from the skin or mucosa.

In the first composition for use in preparation of carbon dioxide gel for external use, it is preferred that the granular material (A) further contains a binder. In this case, when the granular material (A) is mixed with the viscous material (B), the degradation of the granular material (A) is more favorably controlled so that the granular material (A) can be dissolved at a proper rate. Therefore, if the carbon dioxide gel for external use prepared from such a composition for use in preparation of carbon dioxide gel for external use is used, even higher aesthetic or medical effects can be achieved in a more effective manner.

In the first composition for use in preparation of carbon dioxide gel for external use, it is preferred that the viscous material (B) further contains an adhesive for increasing affinity to the skin or mucosa surface. In this case, the mixture of the granular material (A) and the viscous material (B) is more tightly adhered to the skin or mucosa and is even less prone to drop down. This leads to an advantage of more efficient transdermal or transmucosal absorption of carbon dioxide.

In the first composition for use in preparation of carbon dioxide gel for external use, it is preferred that the viscous material (B) further contains an alcohol. In this case, the composition has an advantage of increasing the spreadability of the viscous material (B) and improving the application feeling of the gel.
In the first composition for use in preparation of carbon dioxide gel for external use, it is preferred that the mean particle size of the granular material (A) is defined to range from 0.05 to 1.0 mm. In this case, when the granular material (A) is mixed with the viscous material (B), the granular material (A) can be dissolved at a proper rate so that the end time of the generation/dissolution of carbon dioxide is even more accurately synchronized with the end time of the reaction of the gelling agent.

The second composition for use in preparation of carbon dioxide gel for external use according to the present invention is used for the preparation of a carbon dioxide gel for external use including a gel and carbon dioxide dissolved therein in a substantial non-bubble form and comprises the following granular material (X) and viscous material (Y) to be mixed with the granular material (X):
(X) a granular material comprising a weak acid and a dispersant as essential components;
(Y) a viscous material comprising calcium carbonate, a gelling agent gelated by calcium ions, a calcium ion trapping agent and water as essential components.

The term "dispersant", as used herein, means an agent which acts to disperse ingredients such as the weak acid in the granular material thereby properly retarding the release of the weak acid from the granular material and which, per se, is dissolved or swelled in water at a proper rate.

According to the second composition for use in preparation of carbon dioxide gel for external use, the mixing of the above granular material (X) with the above viscous material (Y) causes the weak acid in the granular material (X) to react with calcium carbonate in the viscous material (Y) so as to generate carbon dioxide, just as in the first composition for use in preparation of carbon dioxide gel for external use. In the meantime, the gelling agent in the viscous material (Y) is gelated by calcium ions released from the above calcium carbonate, and the resultant gel is solidified. In this process, the dispersant acts to retard contact between the weak acid in the granular material (X) dissolved by the viscous material (Y) and calcium carbonate and hence, the generation of carbon dioxide is slowed down. Furthermore, the calcium ion trapping agent traps the calcium ions so as to suppress the rapid progress of the gelation. Therefore, the carbon dioxide gel for external use can be obtained in which a large amount of carbon dioxide in the substantial non-bubble form is dissolved. Such a carbon dioxide gel for external use affords high transdermal or transmucosal absorption of carbon dioxide and hence, even higher aesthetic or medical effects can be achieved in a shorter time. What is more, the composition in the gel form is less prone to drop down on the skin/mucosa. In addition, if the mixing ratios or the like of the ingredients are adjusted, the formed gel can be solidified after the lapse of a predetermined length of time from preparation, so as to be easily pealed off from the skin/mucosa.

In the second composition for use in preparation of carbon dioxide gel for external use, it is preferred that the calcium ion trapping agent of the granular material (X) is disodium hydrogenphosphate. In this case, the calcium ions are more effectively trapped so that the rapid progress of the gelation can be more effectively suppressed. Therefore, when the carbon dioxide gel for external use prepared from such a composition for use in preparation of carbon dioxide gel for external use is used, even higher aesthetic or medical effects can be achieved more effectively.

In the second composition for use in preparation of carbon dioxide gel for external use, it is preferred that the weak acid of the granular material (X) is at least one of sodium dihydrogenphosphate and potassium dihydrogenphosphate, that the gelling agent of the viscous material (Y), which is gelated by the calcium ions, is sodium alginate, and that the calcium ion trapping agent of the viscous material (Y) is disodium hydrogenphosphate. In this case, it is advantageously easy to prepare the carbon dioxide gel for external use having carbon dioxide dissolved therein in the substantial non-bubble form. Furthermore, the end time of the generation/dissolution of carbon dioxide is substantially synchronized with the end time of the reaction of the gelling agent. Thus, the composition has an advantage of ensuring the preparation of the carbon dioxide gel for external use which delivers even higher aesthetic or medical effects in a shorter time. In addition, the composition offers an easy preparation of the carbon dioxide gel for external use which is less prone to drop down on the skin/mucosa. The composition has another advantage that if the mixing ratios or the like of the ingredients are adjusted, a carbon dioxide gel for external use can be readily prepared which is solidified after the lapse of a predetermined length of time from preparation, thus providing for the easy peeling-off thereof.

In the second composition for use in preparation of carbon dioxide gel for external use, it is preferred that the granular material (X) further contains a binder. In this case, when the granular material (X) is mixed with the viscous material (Y), the degradation of the granular material (X) is more favorably controlled by way of the binder used in combination of the dispersant of the granular material (X), so that the granular material (X) can be dissolved at a proper rate. Therefore, when the carbon dioxide gel for external use prepared from such a composition for use in preparation of carbon dioxide gel for external use is used, even higher aesthetic or medical effects can be achieved more effectively.

In the second composition for use in preparation of carbon dioxide gel for external use, it is preferred that the viscous material (Y) further contains an adhesive for increasing affinity to the skin or mucosa surface. In this case, the mixture of the granular material (X) and the viscous material (Y) is more tightly adhered to the skin/mucosa and is less prone to drop down. This leads to an advantage of more efficient transdermal or transmucosal absorption of carbon dioxide in the substantial non-bubble form.
In the second composition for use in preparation of carbon dioxide gel for external use, it is preferred that the viscous material (Y) further contains an alcohol. In this case, the composition has an advantage of increasing the spreadability of the viscous material (Y) and improving the application feeling of the gel.

In the second composition for use in preparation of carbon dioxide gel for external use, it is preferred that the mean particle size of the granular material (X) is defined to range from 0.05 to 1.0 mm. In this case, when the granular material (X) is mixed with the viscous material (Y), the granular material (X) can be dissolved at a proper rate so that the end time of the production/dissolution of carbon dioxide is even more accurately synchronized with the end time of the reaction of the gelling agent.

A carbon dioxide gel for external use according to the present invention is prepared using the aforementioned first or second composition for use in preparation of carbon dioxide gel for external use and is characterized in that carbon dioxide in the substantial non-bubble form is dissolved in the gel. According to the above composition, the gel can contain a large amount of carbon dioxide in the substantial non-bubble form because of the use of the first or second composition for use in preparation of carbon dioxide gel for external use which can control the rates of production/dissolution of carbon dioxide and the rate of reaction of the gelling agent. This leads to an increased amount of transdermally or transmucosally absorbed carbon dioxide so that the higher aesthetic or medical effects can be achieved. Provided that carbon dioxide is present in the same weight content, the carbon dioxide gel for external use of the present invention is smaller in volume and higher in physical strength than a carbon dioxide agent for external use of the same weight which contains carbon dioxide bubbles as an essential component. Therefore, the gel for external use of the present invention is more advantageous in that the gel can be applied to the skin/mucosa in such a small, uniform thickness, providing higher aesthetic or medical effects in terms of dosage. Furthermore, the carbon dioxide gel for external use of the invention has a proper degree of viscosity and adhesiveness so as to be less prone to drop down on an applied surface. Hence, the gel for external use of the present invention can achieve the high aesthetic or medical effects without care of soiling clothes or the like. If the mixing ratios or the like of the ingredients are adjusted, the gel can be solidified after the lapse of a predetermined length of time from preparation, thus providing for the easy peeling-off thereof.

A wound covering material according to the present invention comprises the aforementioned first or second composition for use in preparation of carbon dioxide gel for external use and is characterized in that carbon dioxide in the substantial non-bubble form is dissolved in the gel. According to the above composition, the gel can contain a large amount of carbon dioxide in the substantial non-bubble form because of the use of the first or second composition for use in preparation of carbon dioxide gel for external use which can control the rates of generation/dissolution of carbon dioxide and the rate of reaction of the gelling agent. This leads to an increased amount of transdermally or transmucosally absorbed carbon dioxide so that the higher aesthetic or medical effects can be achieved. If the carbon dioxide gel for external use prepared from the first or second composition for use in preparation of carbon dioxide gel for external use is applied to a wounded area or the like to form a hydrogel sheet and then, is allowed to adhere thereto in an as-is state rather than immediately peeled off from the skin/mucosa, the gel for external use of the present invention can advantageously function as a protection sheet for wounds and the like.

### Best Modes for Carrying Out the Invention

Next, the first composition for use in preparation of carbon dioxide gel for external use according to the present invention and the second composition for use in preparation of carbon dioxide gel for external use according to the present invention will be described discretely.

### First Composition for Use in Preparation of Carbon Dioxide Gel for External Use

The first composition for use in preparation of carbon dioxide gel for external use according to the present invention comprises: a granular material (A) including a weak acid a dispersant and a calcium ion trapping agent as essential components; and a viscous material (B) including calcium carbonate, a gelling agent gelated by calcium ions and water as essential components.

The weak acid constituting the granular material (A) may include inorganic and organic acids whose pH values generally lie in the range of 3.0 (inclusive) to 7.0 (uninclusive) in a 1 wt% aqueous solution. These inorganic and organic acids can be used alone or in combination of plural types. The weak acid is used for the following reasons. In a case where a strong acid is used, even though the acid is used in a small amount, the acid reacts with calcium carbonate so rapidly that a rapid generation of carbon dioxide occurs locally. The resultant carbon dioxide will form bubbles in the gel or will be leak out into the atmosphere. On the other hand, calcium ions will be also released rapidly so that the calcium ion trapping agent will be also unable to fully lower the reaction rate of the gelling agent. Hence, the gelling agent will be quickly gelated and solidified. That is, the weak acid is used for the purpose of dissolving a large amount of carbon dioxide in the substantial non-bubble form in the gel. Above all, preferred weak acids are those which have a buffer action such as to inhibit acidity from exceeding a certain level even when used in high concentrations. More preferred acids are sodium dihdrogenphosphate and potassium dihydrogenphosphate.

In principle, a mixing ratio of the weak acid, which may also vary depending upon the types thereof, may preferably be 5 to 75wt% based on the overall weight of the granular material (A), and more preferably 30 to 50wt%. If the mixing ratio of the weak acid is less than 5wt%, the amount of generated carbon dioxide and the amount of released calcium ions are generally small and hence, too small an amount of carbon dioxide in the non-bubble form may be dissolved in the gel or an insufficient gelation may result. However, such problems may also be associated with the amount of viscous material (B) relative to that of the granular material (A), the ingredients of the viscous material (B) or the proportions thereof. Conversely, if the mixing ratio of the weak acid exceeds 75wt%, the amount of generated carbon dioxide and the amount of released calcium ions are generally excessive and hence, the carbon dioxide may form bubbles in the gel or the gel may be solidified rapidly, terminating the generation of carbon dioxide. However, such problems may also be associated with the amount of viscous material (B) relative to that of the granular material (A), the ingredients of the viscous material (B) or the proportions thereof. That is, it is preferred to limit the mixing ratio of the weak acid to the above range because a large amount of carbon dioxide in the non-bubble form can be dissolved in the gel.

The calcium ion trapping agent constituting the granular material (A) is not particularly limited so long as the agent binds with calcium ions in competition with the gelling agent. Specific examples of a usable calcium ion trapping agent include: phosphate or phosphonate-base calcium ion trapping agents such as disodium hydrogenphosphate, sodium polyphosphate, sodium hexametaphosphate, tetrasodium pyrophosphate, sodium tripolyphosphate, hydroxyethane diphosphonate, hydroxyethylidene diphosphonate, tetrasodium hydroxyethylidene diphosphonate, nitrilotris (methylene phosphonate) and phosphonobutane tricarboxylate; carboxylate-base calcium ion trapping agents such as trisodium citrate and sodium gluconate; aminocarboxylate-base calcium ion trapping agents such as ethylenediamine tetraacetate, disodium ethylenediamine tetraacetate, trisodium ethylenediamine tetraacetate, tetrasodium ethylenediamine tetraacetate, dipotassium ethylenediamine tetraacetate, hydroxyethyl ethylendiamine triacetate, trisodium hydroxiethyl ethylendiamine triacetate, diethylenetriamine pentaacetate, pentasodium diethylenetriamine pentaacetate, calcium disodium ethylenediamine tetraacetate, nitrilotriacetate, methylglycine diacetate, trisodium ethylenediamine hydroxyethyltriacetate, dihydroxyethyl ethylenediamine diacetate, propanediamine tetraacetate, triethylenetetramine hexaacetate, hydroxyethyl iminodiacetate, tetrasodium dicarboxylethyl glutamate, dihydroxyethyl glycine, and 1,3-diamino-2-hydroxypropane tetraacetate; amino-acid-base calcium ion trapping agents such as glycine; and the like. These compunds may be used alone or in combination of plural types. Above all, disodium ethylenediamine tetraacetate and glycine are more preferred because these compounds are excellent in controlling the trapping and re-release of calcium ions or controlling the gelation rate and solidification rate.

In principle, a mixing ratio of the calcium ion trapping agent, which may also vary depending upon the types thereof, may preferably be 0.5 to 50wt% based on the overall weight of the granular material (A) and more preferably 4.0 to 40wt%. If the mixing ratio of this agent is less than 0.5wt%, an insufficient amount of calcium ions is trapped and hence, the gelation and the gel solidification may proceed so rapidly and terminate the generation of carbon dioxide. However, such problems may also be associated with the amount of viscous material (B) relative to that of the granular material (A), the ingredients of the viscous material (B) or the proportions thereof. Conversely, if the mixing ratio exceeds 50wt%, an excessive amount of calcium ions is trapped so that insufficient gelation and gel solidification may result. Hence, the generated carbon dioxide may form bubbles in the gel or may leak out into the atmosphere. However, such problems may also be associated with the amount of viscous material (B) relative to that of the granular material (A), the ingredients of the viscous material (B) or the proportions thereof.

The granular material (A) including the weak acid, a dispersant and the calcium ion trapping agent as the essential components may be in the form of, for example, fine particles or granulated powder. In principle, a mean particle size (a half of the sum of the maximum length and the minimum length of one particle) of the granular material may preferably be defined to range from 0.05 to 1.0 mm and particularly preferably from 0.1 to 0.3 mm. It is preferred that the granular material has a smaller mean particle size than that of a granular material of the conventional composition for use in preparation of carbon dioxide agent for external use (see Publication No.WO 02/80941). If the granular material (A) is too large in size, the granular material (A), as mixed with the viscous material (B), is so slow to be dissolved and thence, the reaction between the weak acid and calcium carbonate tends to be slowed down. This may result in an excessively slow generation of carbon dioxide or insufficient gelation and gel solidification. Conversely, if the granular material (A) is too small in size, the granular material, as mixed with the viscous material (B), is so rapidly dissolved that the reaction between the weak acid and calcium carbonate tends to proceed too rapidly. As a result, carbon dioxide may be generated rapidly, while the generated carbon dioxide may form bubbles in the gel or may leak out into the atmosphere. Furthermore, calcium ions may be released so rapidly that the gelation and gel solidification may proceed rapidly. It is noted that the granular material (A) may preferably have a rougher surface than a mirror surface because the granular material may be more uniformly degraded and dissolved as placed in contact with the viscous material (B).

The granular material (A) can be prepared by any of the known methods such as wet granulation process and dry granulation process, which has, in addition to the above components, a dispersant for preventing the reaction between the weak acid and calcium carbonate from proceeding too rapidly, and which may use, in addition to the above essential components, a binder for maintaining the shape of particles or the like. The additive may be used as required.

The dispersant is not particularly limited and may be any fine particles that are relatively freely dissolved or swelled as mixed with the viscous material (B) and are able to disperse the other ingredients. Examples of a usable dispersant include: starch derivatives such as pregelatinized starch and a-cyclodexitrin; saccharides such as white sugar, glucose, fructose, sucrose, lactose, xylitol, D-sorbitol and D-mannitol; polysaccharides such as pullulan and xanthan gum; cellulose derivatives and salts thereof such as hydroxypropyl cellulose, hydroxypropylmethyl cellulose, carmellose calcium and carmellose sodium; synthetic macromolecules such as polyvinylpyrrolidone; and urea. These compounds can be used alone or in combination of plural types.

The binder is not particularly limited and may be any fine particles that are used as dissolved or swelled in a suitable solvent and are able to bind two or more substances. Examples of a usable binder include: gum arabic, crystalline cellulose, carmellose sodium, methyl cellulose, hydroxypropyl cellulose, hydroxypropylmethyl cellulose, hydroxypropylmethyl cellulose terephthalate, sodium carboxymethyl cellulose, carboxymethylethyl cellulose, hydroxyethyl cellulose, dextrin, wheat starch, rice starch, corn starch, hydroxypropyl starch, pregelatinized starch, pullulan, polyvinylpyrrolidone, methacrylic copolymer, polyvinyl alcohol, agar, gelatin, gum tragacanth, potato starch and the like. These compounds may be used alone or in combination of plural types.

It is preferred that the weak acid, the dispersant and the calcium ion trapping agent are dispersed as homogeneously as possible in the granular material (A) prepared in this manner. When such a granular material (A) and the viscous material (B) are homogeneously blended together, local increase or decrease of calcium ion concentration is suppressed, while excessive local increase or decrease of carbon dioxide generation is also suppressed. That is, there may be obtained a carbon dioxide gel for external use, in the whole mass of which a sufficient amount of carbon dioxide in the non-bubble form is dissolved.

In a case where the reaction between the weak acid and calcium carbonate is too rapid, the granular material (A) may be coated with a retarding material (a material acting to lower the rate at which the granular material is dissolved in the viscous material (B)) so as to form a release controlling granular material which is able to control the release of the weak acid or the like. Otherwise, the binder may be used for the production of the granular material (A) so as to form a degradation-rate controllable granular material which is adapted to lower the rate of degradation of the granular material (A).

On the other hand, the viscous material (B) to be used in combination with the above granular material (A) includes calcium carbonate as an essential component. Calcium carbonate is used as the carbonate for the following reason. In a case where magnesium carbonate containing a divalent metal ion similarly to calcium carbonate is used as the carbonate, magnesium carbonate naturally generates carbon dioxide as reacting with the acid. In a case where magnesium carbonate is used to gelate the gelling agent, however, the resultant gel tends to be decreased in hardness. Accordingly, the carbon dioxide, as generated in the gel, readily forms bubbles in the gel or readily leaks out into the atmosphere. This makes it difficult to obtain a carbon dioxide gel for external use in which a sufficient amount of carbon dioxide in the substantial non-bubble form is dissolved. On this account, calcium carbonate is used as the essential component which is responsible for the generation of carbon dioxide and the release of the divalent metal ions.

In principle, a mixing ratio of calcium carbonate, which may also vary depending upon the types of the weak acid and gelling agent or the mixing ratio thereof, may preferably be 0.1 to 6.0wt% based on the overall weight of the viscous material (B), more preferably 0.2 to 2.0wt% and even more preferably 0.3 to 1.0wt%. If the mixing ratio of calcium carbonate is less than 0.1 wt%, the generation of carbon dioxide may be too low or an insufficient gelation may result. However, such problems may also be associated with the amount of granular material (A) relative to that of the viscous material (B), the ingredients of the granular material (A) or the proportions thereof. Conversely, if the mixing ratio exceeds 6.0wt%, the viscous material (B), as mixed with the granular material (A), may cause such rapid generation and dissolution of carbon dioxide that the generated carbon dioxide may form bubbles in the gel or may leak out into the atmosphere. However, such problems may also be associated with the amount of granular material (A) relative to that of the viscous material (B), the ingredients of the granular material (A) or the proportions thereof.

The gelling agent constituting the viscous material (B) is not particularly limited and can be any material that is capable of being gelated by calcium ions released from calcium carbonate. Examples of a usable gelling agent include: carrageenan, low methoxylated pectin, sodium alginate, potassium alginate, ammonium alginate and the like. These compounds can be used alone or in combination of plural types. Above all, sodium alginate is preferred because of excellent affinity to the skin/mucosa and good application feelimg.

In principle, a mixing ratio of the gelling agent, which may also vary depending upon the types thereof, may preferably be 0.3 to 15.0wt% based on the overall weight of the viscous material (B), more preferably 0.3 to 8.0wt% and even more preferably 1.5 to 5.0wt%. If the mixing ratio of the gelling agent is less than 0.3wt%, the viscous material (B), as mixed with the granular material (A), may encounter an insufficient gelation and hence, the generated carbon dioxide may form bubbles in the gel or may leak out into the atmosphere. However, such problems may also be associated with the amount of granular material (A) relative to that of the viscous material (B), the ingredients of the granular material (A), or the proportions thereof. Conversely, if the mixing ratio exceeds 15.0wt%, the viscous material (B), as mixed with the granular material (A), may be excessively increased in viscosity so as to become unable to further dissolve the granular material (A). Hence, the reaction between the weak acid and calcium carbonate may not proceed any further. However, such problems may also be associated with the amount of granular material (A) relative to that of the viscous material (B), the ingredients of the granular material (A), or the proportions thereof.

The water constituting the viscous material (B) is not particularly limited and can be natural water, tap water, distilled water, purified water or the like. Carbon dioxide is soluble not only in water but also in a variety of solvents such as organic solvents and lipids. Above all, water facilitates the reaction between the weak acid and calcium carbonate better than the organic solvents and lipids. Furthermore, water also facilitates the generation and dissolution of carbon dioxide, the release of calcium ions and the gelation of the gelling agent better than the organic solvents and lipids. What is more, water is generally easy to use, less costly and safe. On this account, water is used as the essential component.

In principle, a mixing ratio of water, which may also vary depending upon the types of the other ingredients or the mixing ratios thereof, may preferably be 70 to 95wt% based on the overall weight of the viscous material (B). If the mixing ratio of water is less than 70wt%, the granular material (A) is slightly soluble in water so that the reaction between the weak acid and calcium carbonate is less likely to be effected. Conversely, if the mixing ratio exceeds 95wt%, the mixing ratios of the other essential components are too small. When the viscous material (B) is mixed with the granular material (A), therefore, insufficient gelation, insufficient gel solidification or insufficient generation of carbon dioxide may result.

It is preferred that the viscous material (B) essentially comprising calcium carbonate, the gelling agent and water has such a viscosity as to allow the granular material (A), as admixed thereto, to be readily and homogeneously dispersed in the viscous material. In a case where the viscous material (B) is mixed with the granular material (A) and the resultant mixture is immediately applied directly onto the skin or mucosa, it is preferred that the viscous material has such a viscosity as to prevent the mixture from dropping down on the skin or mucosa just after the application.

The viscous material (B) may be prepared by adding an adhesive for increasing the affinity to the skin or mucosa surface (hereinafter, simply referred to as "adhesive") and the like to the above essential components and blending together the essential components, the adhesive and the like. The adhesive and the like may be added as required.
Examples of a usable adhesive include: gum Arabic, crystalline cellulose, carmellose sodium, methyl cellulose, hydroxypropyl cellulose, hydroxypropylmethyl cellulose, hydroxypropylmethyl cellulose terephthalate, sodium carboxymethyl cellulose, carboxymethylethyl cellulose, hydroxyethyl cellulose, dextrin, wheat starch, rice starch, corn starch, hydroxypropyl starch, pregelatinized starch, pullulan, polyvinylpyrrolidone, methacrylic copolymer, polyvinyl alcohol, gelatin, gum tragacanth, potato starch, agar and the like. These compounds can be used alone or in combination of plural types.

In principle, a mixing ratio of the adhesive, which may also vary depending upon the types thereof, may preferably be 0.1 to 5.0wt% based on the overall weight of the viscous material (B). If the mixing ratio of the adhesive is less than 0.1 wt%, the adhesive exhibits an insufficient adhesive power and hence, there is little point in using the adhesive. Conversely, if the mixing ratio exceeds 5.0wt%, the adhesive may exhibit too strong an adhesive power and hence, it may become difficult to remove the applied gel from the skin/mucosa.

The first composition for use in preparation of carbon dioxide gel for external use, which comprises the above granular material (A) and viscous material (B), may be used as follows, for example. First, the granular material (A) and the viscous material (B) are placed in any of various vessels and blended together in a predetermined mixing ratio. It is preferred that the mixing ratio of these materials may be so defined as to allow calcium carbonate to react with the all amount of weak acid. However, it is unfavorable that the granular material (A) is used in such a great amount relative to the viscous material (B) that calcium carbonate and the weak acid react so rapidly as to effect rapid gelation and gel solidification, or that carbon dioxide is generated so rapidly as to form bubbles in the gel or to leak out into the atmosphere. More specifically, a molar ratio between the weak acid of the granular material (A) and the calcium carbonate of the viscous material (B) is preferably on the order of 0.4 to 40 in a case where a mixing ratio of calcium carbonate is 0.1wt%; preferably on the order of 0.04 to 3.6 in a case where a mixing ratio of calcium carbonate is 0.3wt%; and preferably on the order of 0.02 to 0. 54 in a case where a mixing ratio of calcium carbonate is 6.0wt%. Immediately after the preparation, the resultant mixture is applied to a predetermined area of the skin or mucosa. At this time, there is formed a carbon dioxide gel for external use wherein carbon dioxide generated in the reaction between the weak acid and calcium carbonate is dissolved in the gel as being in the substantial non-bubble form. The carbon dioxide is transdermally or transmucosally absorbed in the skin or mucosa, so as to deliver high aesthetic or medical effects. If the mixing ratios of the ingredients are adjusted, the gel is solidified after the lapse of a predetermined length of time from preparation and hence, the gel can be easily peeled off from the skin or mucosa.

So long as the effect of the present invention is not impaired, at least one of the granular material (A) and the viscous material (B) may be admixed with a material generally used in agents for external use and cosmetics, whenever necessary. Examples of such a material include: fragrances, colorants, surfactants, oils, moisturizers, thickeners, alcohols, preservatives, antioxidants, anticoloring agents, UV absorbing/scattering agents, drugs and the like. The admixture of such a material allows the resultant carbon dioxide gel for external use to be used as an even more favorable type of cosmetic or medicinal product for external use. It is particularly preferred to use an alcohol in the viscous material (B) because the alcohol increases the spreadability of the gel and improves the application feeling. It is also preferred to use a thickener in at least one of the granular material (A) and the viscous material (B) because a thickener increases the viscosity and adhesiveness of the gel.

Examples of a usable alcohol include: monohydric alcohols such as ethanol, propanol, isopropanol and butanol; and polyhydric alcohols such as 1,3-butylene glycol, xylitol, glycerol, sorbitol, propylene glycol, dipropylene glycol, hexylene glycol, pentylene glycol and polyethylene glycol. These alcohols can be used alone or in combination of plural types. Above all, 1,3-butylene glycol, propylene glycol, pentylene glycol, glycerol and polyethylene glycol are more preferred.

The following natural macromolecules, semisynthetic macromolecules, synthetic macromolecules and inorganic substances can be used as the thickener. These materials can be used alone or in combination of plural types.
Natural macromolecules: plant derived macromolecules such as gum Arabic, galactan, agar, quince seed gum, guar gum, gum tragacanth, pectin, mannan, locust bean gum, rice starch, wheat starch, corn starch and potato starch; microorganism derived macromolecules such as Curdlan, xanthan gum, succinoglucan, dextran, hyaluronic acid and pullulan; and protein derived macromolecules such as albumin, casein, collagen, gelatin and fibroin.

Semisynthetic macromolecules: cellulose derived macromolecules such as ethyl cellulose, processed starch, carboxymethyl cellulose and salts thereof, carboxymethylethyl cellulose and salts thereof, carboxymethyl starch and salts thereof, croscarmellose and salts thereof, crystalline cellulose, cellulose acetate, cellulose acetate phthalate, hydroxyethyl cellulose, hydroxypropyl starch, hydroxypropyl cellulose, hydroxypropylmethyl cellulose, hydroxypropylmethyl cellulose phthalate, cellulose powder, methyl cellulose and methylhydroxypropyl cellulose; starch derived macromolecules such as pregelatinized starch, partly pregelatinized starch, carboxymethyl starch, dextrin and methyl starch; and other polysaccharide derived macromolecules such as sodium condroitin sulfate and sodium hyaluronate.

Synthetic macromolecules: carboxyvinyl polymer, sodium polyacrylate, polyvinylacetal diethylaminoacetate, polyvinyl alcohol, polyvinylpyrrolidone, methacrylic acid-ethyl acrylate copolymer, methacrylic acid-ethyl methacrylate copolymer, ethyl methacrylate-trimethylammonium chloride ethyl methacrylate copolymer, dimethylaminoethyl methacylate-methyl methacrylate copolymer and the like.
Inorganic substances: hydrated silicon dioxide, light anhydrous silicic acid, colloidal alumina, bentonite, laponite and the like.

As to the composition for use in preparation of carbon dioxide gel for external use according to the best mode of the present invention, the granular material (A) and the viscous material (B) may be stored in a practically out-of-contact state until use and may preferably be stored in a sealed state. Any types of storage containers are usable with no particular restriction posed on the material, shape or structure. Examples of a usable material include: plastics, glass, aluminum, paper, various types of polymers and composites thereof. Examples of available shape and structure of the container include cups, tubes, bags, bottles, sticks and dispensers.

### Second Composition for Use in Preparation of Carbon Dioxide Gel for External Use

The second composition for use in preparation of carbon dioxide gel for external use according to the present invention comprises: a granular material (X) including a weak acid and a dispersant as essential components; and a viscous material (Y) including calcium carbonate, a gelling agent gelated by calcium ions, a calcium ion trapping agent and water as essential components. The composition differs from the first composition for use in preparation of carbon dioxide gel for external use in that the calcium ion trapping agent is used as the essential component of the viscous material (Y) rather than of the granular material (X), and that the dispersant is used as the essential component of the granular material (X). Such a composition is made for the following reasons. In a case where the viscous material (Y) contains the calcium ion trapping agent, when the viscous material (Y) is mixed with the granular material (X) including the weak acid, a rapid reaction between the weak acid and calcium carbonate takes place rapidly before the weak acid is dispersed in the overall viscous material (Y). Hence, the gelation and the gel solidification may take place locally, or the generated carbon dioxide may be locally distributed.

Similarly to that of the first composition for use in preparation of carbon dioxide gel for external use, the weak acid constituting the granular material (X) can include inorganic and organic acids whose pH values generally lie in the range of 3.0 (inclusive) to 7.0 (uninclusive) in an 1wt% aqueous solution. These acids can be used alone or in combination of plural types. The weak acid is used for the same reason as that stated in the first composition for use in preparation of carbon dioxide gel for external use. Above all, preferred weak acids are those which have a buffer action such as to inhibit acidity from exceeding a certain level even when used in high concentrations. More preferred acids are sodium dihydrogenphosphate and potassium dihydrogenphosphate.

In principle, a mixing ratio of the weak acid, which may also vary depending upon the types thereof, may preferably be 10 to 75wt% based on the overall weight of the granular material (X), more preferably 10 to 60wt% and even more preferably 20 to 50wt%. That is, the mixing ratio of the weak acid may preferably be lower than that of the first composition for use in preparation of carbon dioxide gel for external use. The reason for limiting the mixing ratio to the above range is as follows. If the mixing ratio of the weak acid is less than 10wt%, the amount of generated carbon dioxide and the amount of released calcium ions are generally small and hence, an excessively low amount of carbon dioxide in the non-bubble form may be dissolved in the gel or an insufficient gelation may result. However, such problems may also be associated with the amount of viscous material (Y) relative to that of the granular material (X), the ingredients of the viscous material (Y) or the proportions thereof. Conversely, if the mixing ratio exceeds 75wt%, the amount of generated carbon dioxide and the amount of released calcium ions are generally excessive so that the carbon dioxide may form bubbles in the gel or the gel may be solidified so rapidly as to terminate the generation of carbon dioxide. However, such problems may also be associated with the amount of viscous material (Y) relative to that of the granular material (X), the ingredients of the viscous material (Y) or the proportions thereof. That is, it is preferred to limit the mixing ratio of the weak acid to the above range because a large amount of carbon dioxide in the non-bubble form can be dissolved in the gel.

The dispersant constituting the granular material (X) is not particularly limited and can be any fine particles that are relatively freely dissolved or swelled as mixed with the viscous material (Y) and are able to disperse the other ingredients. It is noted here that the dispersant is used as the essential component of the granular material (X) in order to prevent too early contact between the weak acid in the granular material (X) free from the calcium ion trapping agent and the calcium carbonate in the viscous material (Y), in which the granular material (X) is dissolved. Examples of a usable dispersant include: starch derivatives such as pregelatinaized starch and α-cyclodexitrin; saccharides such as white sugar, glucose, fructose, sucrose, lactose, xylitol, D-sorbitol and D-mannitol; polysaccharides such as pullulan and xanthan gum; cellulose derivatives and salts thereof such as hydroxypropyl cellulose, hydroxypropylmethyl cellulose, carmellose calcium and carmellose sodium; synthetic macromolecules such as polyvinylpyrrolidone; and urea. These compounds can be used alone or in combination of plural types.

In principle, a mixing ratio of the dispersant, which may also vary depending upon the types or the mixing ratio of the weak acid, may preferably be 10 to 90wt% based on the overall weight of the granular material (X) and more preferably 20 to 40wt%. If the mixing ratio of the dispersant is less than 10wt%, when the granular material is mixed with the viscous material (Y), the local concentration of the weak acid is so high as to react rapidly with calcium carbonate at local places in the viscous material (Y) and to stop reacting. Consequently, the whole gelling mass may not be gelated. However, such problems may also be associated with the amount of viscous material (Y) relative to that of the granular material (X), the ingredients of the viscous material (Y) or the proportions thereof. Conversely, if the mixing ratio exceeds 90wt%, when the granular material is mixed with the viscous material (Y), the local concentration of the weak acid is so low that the amount of carbon dioxide generated in the reaction with calcium carbonate and the amount of released calcium ions are insufficient. Therefore, a sufficient amount of carbon dioxide in the non-bubble form may not be dissolved in the gel, or a retarded gelation process may detrimentally allow the generated carbon dioxide to form bubbles in the gel or to leak out into the atmosphere. However, such problems may also be associated with the amount of viscous material (Y) relative to that of the granular material (X), the ingredients of the viscous material (Y) or the proportions thereof.

The granular material (X) including the weak acid and the dispersant as the essential components may be in the form of, for example, fine particles or granulated powder. In principle, a mean particle size (a half of the sum of the maximum length and the minimum length of one particle) of the granular material may preferably be defined to range from 0.05 to 1.0 mm and particularly preferably 0.1 to 0.3 mm. It is preferred that the granular material has a smaller mean particle size than that of the granular material of the conventional composition for use in preparation of carbon dioxide agent for external use (see Publication No.WO 02/80941). If the granular material (X) is too large in size, the granular material (X), as mixed with the viscous material (Y), is so slow to be dissolved and thence, the reaction between the weak acid and calcium carbonate tends to be slowed down. This may result in the excessively slow generation of carbon dioxide or the insufficient gelation and gel solidification. Conversely, if the granular material (X) is too small in size, the granular material, as mixed with the viscous material (Y), is so rapidly dissolved that the reaction between the weak acid and calcium carbonate tends to proceed too rapidly. As a result, carbon dioxide may be generated rapidly, while the generated carbon dioxide may form bubbles in the gel or may leak out into the atmosphere. Furthermore, calcium ions may be released so rapidly that the gelation and gel solidification may proceed rapidly. It is noted that granular material (X) may preferably have a rougher surface than a mirror surface because the granular material may be more uniformly degraded and dissolved as placed in contact with the viscous material (Y).

The granular material (X) can be prepared by any of the known methods such as wet granulation process and dry granulation process, which can use, in addition to the above essential components, a binder for maintaining the shape of particles. The additives may be used as required. The binder may include similar compounds to those used in the first composition for use in preparation of carbon dioxide gel for external use.

It is preferred that the weak acid and the dispersant are dispersed as homogeneously as possible in the granular material (X) prepared in this manner. When such a granular material (X) and the viscous material (Y) are homogeneously blended together, the concentration of calcium ions is prevented from being increased or decreased locally, while the generation of carbon dioxide is also prevented from being increased or decreased locally. That is, there may be obtained a carbon dioxide gel for external use, in the whole mass of which a sufficient amount of carbon dioxide in the non-bubble form is dissolved.

In a case where the reaction between the weak acid and calcium carbonate is too rapid, the granular material (X) may be coated with a retarding material (a material acting to lower the rate at which the granular material is dissolved in the viscous material (Y)) so as to form a release controlling granular material which is able to control the release of the weak acid or the like. Otherwise, the binder may be used in the production of the granular material (X) so as to form a degradation-rate controlling granular material which is adapted to lower the rate of degradation of the granular material (X).

On the other hand, the viscous material (Y) to be used in combination with the above granular material (X) includes calcium carbonate as an essential component. Calcium carbonate is used as the carbonate for the same reason as that stated in the first composition for use in preparation of carbon dioxide gel for external use. In principle, a mixing ratio of calcium carbonate, which may also vary depending upon the types of the weak acid and gelling agent or the mixing ratios thereof, may preferably be 0.1 to 6.0wt% based on the overall weight of the viscous material (Y), more preferably 0.2 to 2.0wt% and even more preferably 0.3 to 1.0wt%. If the mixing ratio of calcium carbonate is less than 0.1wt%, the generation of carbon dioxide may be too low or an insufficient gelation may result. However, such problems may also be associated with the amount of granular material (X) relative to that of the viscous material (Y), the ingredients of the granular material (X), or the proportions thereof. Conversely, if the mixing ratio exceeds 6.0wt%, the viscous material (Y), as mixed with the granular material (X), may cause such rapid generation and dissolution of carbon dioxide that the generated carbon dioxide may form bubbles in the gel or may leak out into the atmosphere. However, such problems may also be associated with the amount of granular material (X) relative to the viscous material (Y), the ingredients of the granular material (X), or the proportions thereof.

Similarly to the first composition for use in preparation of carbon dioxide gel for external use, the gelling agent constituting the viscous material (Y) is not particularly limited and can be any material that is capable of being gelated by calcium ions released from calcium carbonate. Examples of a usable gelling agent include: carrageenan, low methoxylated pectin, sodium alginate, potassium alginate, ammonium alginate and the like. These compounds can be used alone or in combination of plural types. Above all, sodium alginate is preferred because of excellent affinity to the skin/mucosa and good application feeling.

In principle, a mixing ratio of the gelling agent, which may also vary depending upon the types thereof, may preferably be 0.3 to 15.0wt% based on the overall weight of the viscous material (Y), more preferably 0.3 to 8.0wt% and even more preferably 1.5 to 5.0wt%. If the mixing ratio of the gelling agent is less than 0.3wt%, the viscous material (Y), as mixed with the granular material (X), may encounter an insufficient gelation and hence, the generated carbon dioxide may form bubbles in the gel or may leak out into the atmosphere. However, such problems may also be associated with the amount of granular material (X) relative to that of the viscous material (Y), the ingredients of the granular material (X), or the proportions thereof. Conversely, if the mixing ratio exceeds 15.0wt%, the viscous material (Y), as mixed with the granular material (X), may be excessively increased in viscosity so as to become unable to further dissolve the granular material (X). Hence, the reaction between the weak acid and calcium carbonate may not proceed any further. However, such problems may also be associated with the amount of granular material (X) relative to that of the viscous material (Y), the ingredients of the granular material (X), or the proportions thereof.

The calcium ion trapping agent constituting the viscous material (Y) may include those which bind with calcium ions in competition with the gelling agent and which are alkaline or neutral. In the first composition for use in preparation of carbon dioxide gel for external use, the calcium ion trapping agent is contained in the granular material (A). Therefore, even if an acidic agent is used, the agent does not involve a care of reacting with calcium carbonate to generate carbon dioxide during storage. According to the present embodiment, however, the calcium ion trapping agent is contained in the viscous material (Y). Hence, if the agent is acidic, the agent reacts with calcium carbonate to generate carbon dioxide during the preparation of the viscous material (Y). On this account, the present embodiment employs the calcium ion trapping agent which binds with calcium ions in competition with the gelling agent and which is alkaline or neutral. Examples of a usable calcium ion trapping agent include: sodium hexametaphosphate, tetrasodium pyrophosphate, disodium hydrogenphosphate, trisodium citrate, sodium polycarboxylate, tetrasodium hydroxyethylidene diphosphonate, trisodium ethylenediamine tetraacetate, tetrasodium ethylenediamine tetraacetate, sodium hydroxyethylethylenediamine triacetate, pentasodium diethylenetriamine pentaacetate, calcium disodium ethylenediamine tetraacetate and the like. These compunds can be used alone or in combination of plural types. Above all, disodium hydrogenphosphate is preferred.

In principle, a mixing ratio of the calcium ion trapping agent, which may also vary depending upon the types thereof, may preferably be 0.1 to 1.0wt% based on the overall weight of the viscous material (Y). If the mixing ratio of the calcium ion trapping agent is less than 0.1wt%, an insufficient amount of calcium ions is trapped and hence, the gelation and the gel solidification proceed so rapidly as to terminate the generate of carbon dioxide. However, such problems may also be associated with the amount of granular material (X) relative to that of the viscous material (Y), the ingredients of the granular material (X) or the proportions thereof. Conversely, if the mixing ratio exceeds 1.0wt%, an excessive amount of calcium ions is trapped so that insufficient gelation and gel solidification may result. Hence, the generated carbon dioxide may form bubbles in the gel or may leak out into the atmosphere. However, such problems may also be associated with the amount of granular material (X) relative to that of the viscous material (Y), the ingredients of the granular material (X) or the proportions thereof.

The water constituting the viscous material (Y) is not particularly limited and can be natural water, tap water, distilled water, purified water or the like. Water is used as the essential component for the same reason as that stated in the first composition for use in preparation of carbon dioxide gel for external use.
In principle, a mixing ratio of water, which may also vary depending upon the types of the other ingredients or the mixing ratios thereof, may preferably be 70 to 95wt% based on the overall weight of the viscous material (Y). If the mixing ratio of water is less than 70wt%, the granular material (X) is slightly soluble in water so that the reaction between the weak acid and calcium carbonate is less likely to be effected. Conversely, if the mixing ratio exceeds 95wt%, the mixing ratios of the other essential components are too small. When the viscous material (Y) is mixed with the granular material (X), therefore, insufficient gelation, insufficient gel solidification or insufficient generation of carbon dioxide may result.

It is preferred that the viscous material (Y) essentially including calcium carbonate, the gelling agent, the calcium ion trapping agent and water has such a viscosity as to allow the granular material (X), as admixed thereto, to be readily and homogeneously dispersed in the viscous material. In a case where the viscous material (Y) is mixed with the granular material (X) and the resultant mixture is immediately applied directly onto the skin or mucosa, it is preferred that the viscous material has such a viscosity as to prevent the mixture from dropping down from the skin or mucosa just after the application.

The viscous material (Y) can be prepared by adding an adhesive for increasing the affinity to the skin or mucosa surface and the like to the above essential components and blending together the essential components, the adhesive and the like. The adhesive and the like may be added as required. The types and the mixing ratio of the adhesive are the same as those stated in the first composition for use in preparation of carbon dioxide gel for external use.

The second composition for use in preparation of carbon dioxide gel for external use, which comprises the above granular material (X) and the viscous material (Y), may be used as follows, for example. First, the granular material (X) and the viscous material (Y) are placed in any of various vessels and blended together in a predetermined mixing ratio. It is preferred that the mixing ratio of these materials may be so defined as to allow calcium carbonate to react with the all amount of weak acid. However, it is unfavorable that the granular material (X) is used in such a great amount relative to the viscous material (Y) that calcium carbonate and the weak acid react so rapidly as to effect rapid gelation and gel solidification, or that carbon dioxide is generated so rapidly as to form bubbles in the gel or to leak out into the atmosphere. More specifically, a molar ratio between the weak acid of the granular material (X) and the calcium carbonate of the viscous material (Y) is preferably on the order of 0.4 to 40 in a case where the mixing ratio of calcium carbonate is 0.1 wt%; preferably on the order of 0.04 to 3.6 in a case where the mixing ratio of calcium carbonate is 0.3wt%; and preferably on the order of 0.02 to 0. 54 in a case where the mixing ratio of calcium carbonate is 6.0wt%. Immediately after the preparation, the resultant mixture is applied to a predetermined area of the skin or mucosa. At this time, there is formed a carbon dioxide gel for external use wherein carbon dioxide generated in the reaction between the weak acid and calcium carbonate is dissolved in the gel as being in the substantial non-bubble form. The carbon dioxide is transdermally or transmucosally absorbed in the skin or mucosa, so as to deliver high aesthetic or medical effects. When the mixing ratios of the ingredients are adjusted, the gel is solidified after the lapse of a predetermined length of time from preparation and hence, the gel may be easily peeled off from the skin or mucosa.

So long as the effect of the invention is not impaired, at least one of the granular material (X) and the viscous material (Y) can be admixed with a material generally used in agents for external use and cosmetics, whenever necessary. Examples of such a material include: fragrances, colorants, surfactants, oils, moisturizers, thickeners, alcohols, preservatives, antioxidants, anticoloring agents, UV absorbing/scattering agents, drugs and the like. The admixture of such a material allows the resultant carbon dioxide gel for external use to be used as an even more favorable type of cosmetic or medicinal product for external use. It is particularly preferred to use an alcohol in the viscous material (Y) because the alcohol increases the spreadability of the gel and improves the application feeling. It is also preferred to use a thickener in at least one of the granular material (X) and the viscous material (Y) because the thickener increases the viscosity and adhesiveness of the gel. The usable alcohols and thickeners are the same as those cited in the first composition for use in preparation of carbon dioxide gel for external use.

As to the composition for use in preparation of carbon dioxide gel for external use according to the present invention, the granular material (X) and the viscous material (Y) may be stored in a practically out-of-contact state until use and are preferably stored in a sealed state. Any types of storage containers are usable with no particular restriction posed on the material, shape or structure. Examples of a usable material include: plastics, glass, aluminum, paper, various types of polymers and composites thereof. Examples of available shape and structure include cups, tubes, bags, bottles, sticks and dispensers.

Next, the composition for use in preparation of carbon dioxide gel for external use will be specifically described with reference the examples thereof. It is to be noted that the present invention is not limited to the following examples. Hereinafter, a term "parts by weight" will be abbreviated as "parts".

The following Examples 1 to 5 illustrate the first composition for use in preparation of carbon dioxide gel for external use.

### Example 1 not exactly according to the invention

### Preparation of Granular Material (A)

A granular material (mean particle size: 0.3 mm) was prepared using 57 parts of sodium dihydrogenphosphate as the weak acid, 43 parts of disodium ethylenediamine tetraacetate as the calcium ion trapping agent, and a suitable amount of water.

### Preparation of Viscous Material (B)

A viscous material (B) was prepared using 1 part of calcium carbonate, 3.5 parts of sodium alginate as the gelling agent gelated by calcium ions, 87.9 parts of purified water as the water, 0.9 parts of sodium carboxymethyl cellulose as the adhesive for increasing affinity to the skin/mucosa surface, 3.5 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 2

### Preparation of Granular Material (A)

A granular material (mean particle size: 0.2 mm) was prepared using 53 parts of sodium dihydrogenphosphate as the weak acid, 6 parts of disodium ethylenediamine tetraacetate as the calcium ion trapping agent, 36 parts of lactose as the dispersant, 5 parts of dextrin as the binder and a suitable amount of water.

### Preparation of Viscous Material (B)

A viscous material (B) was prepared using 0.4 parts of calcium carbonate, 3.5 parts of sodium alginate as the gelling agent gelated by calcium ions, 86 parts of purified water as the water, 0.9 parts of sodium carboxymethyl cellulose as the adhesive for increasing affinity to the skin/mucosa surface, 6 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 3

### Preparation of Granular Material (A)

A granular material (mean particle size: 0.3 mm) was prepared using 56 parts of sodium dihydrogenphosphate as the weak acid, 6 parts of disodium ethylenediamine tetraacetate as the calcium ion trapping agent, 33 parts of lactose as the dispersant, 5 parts of dextrin as the binder and a suitable amount of water.

### Preparation of Viscous Material (B)

A viscous material (B) was prepared using 0.3 parts of calcium carbonate, 3.5 parts of sodium alginate as the gelling agent gelated by calcium ions, 85.1 parts of purified water as the water, 0.9 parts of sodium carboxymethyl cellulose as the adhesive for increasing affinity to the skin/mucosa surface, 7 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 4 not exactly according to the invention

### Preparation of Granular Material (A)

A granular material (mean particle size: 0.3 mm) was prepared using 65 parts of sodium dihydrogenphosphate as the weak acid,35 parts of disodium ethylenediamine tetraacetate as the calcium ion trapping agent and a suitable amount of water.

### Preparation of Viscous Material (B)

A viscous material (B) was prepared using 1.0 part of calcium carbonate, 3.5 parts of sodium alginate as the gelling agent gelated by calcium ions, 87.9 parts of purified water as the water, 3.5 parts of 1,3-butylene glycol as the alcohol, 0.9 parts of sodium carboxymethyl cellulose as the adhesive for increasing affinity to the skin/mucosa surface, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 5 not exactly according to the invention

### Preparation of Granular Material (A)

A granular material (mean particle size: 0.2 mm) was prepared using 60 parts of sodium dihydrogenphosphate and 5 parts of potassium dihydrogenphosphate as the weak acid, 35 parts of disodium ethylenediamine tetraacetate as the calcium ion trapping agent and a suitable amount of water.

### Preparation of Viscous Material (B)

A viscous material (B) was prepared using 1.0 part of calcium carbonate, 3.5 parts of sodium alginate as the gelling agent gelated by calcium ions, 87.9 parts of purified water as the water, 3.5 parts of 1,3-butylene glycol as the alcohol, 0.9 parts of sodium carboxymethyl cellulose as the adhesive for increasing affinity to the skin/mucosa surface, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

The following Examples 6 to 25 illustrate the first composition for use in preparation of carbon dioxide gel for external use. These examples use sodium dihydrogenphosphate as the weak acid of the granular material (A) and disodium ethylenediamine tetraacetate as the calcium ion trapping agent of the granular material (A), as principally varying the mixing ratios thereof.

### Example 6

### Preparation of Granular Material (A)

A granular material (mean particle size: 0.3 mm) was prepared using 23.1 parts of sodium dihydrogenphosphate as the weak acid, 3.0 parts of disodium ethylenediamine tetraacetate as the calcium ion trapping agent, 5.0 parts of dextrin as the binder, 68.9 parts of lactose as the dispersant and a suitable amount of 50% ethanol.

### Preparation of Viscous Material (B)

A viscous material (B) was prepared using 0.3 parts of calcium carbonate, 3.5 parts of sodium alginate as the gelling agent gelated by calcium ions, 85.1 parts of purified water as the water, 0.9 parts of sodium carboxymethyl cellulose as the adhesive for increasing affinity to the skin/mucosa surface, 7.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 7

### Preparation of Granular Material (A)

A granular material (mean particle size: 0.3 mm) was prepared using 30.8 parts of sodium dihydrogenphosphate as the weak acid, 5.0 parts of disodium ethylenediamine tetraacetate as the calcium ion trapping agent, 5.0 parts of dextrin as the binding agent, 59.2 parts of lactose as the dispersant and a suitable amount of 50% ethanol.

### Preparation of Viscous Material (B)

A viscous material (B) was prepared the same way as in Example 6.
The resultant granular material (A) and viscous material (B) were paired to provide a composition for use in preparation of carbon dioxide gel for external use.

### Example 8

### Preparation of Granular Material (A)

A granular material (mean particle size: 0.3 mm) was prepared using 43.1 parts of sodium dihydrogenphosphate as the weak acid, 6.0 parts of disodium ethylenediamine tetraacetate as the calcium ion trapping agent, 5.0 parts of dextrin as the binding agent, 45.9 parts of lactose as the dispersant and a suitable amount of 50% ethanol.

### Preparation of Viscous Material (B)

A viscous material (B) was prepared the same way as in Example 6.
The resultant granular material (A) and viscous material (B) were paired to provide a composition for use in preparation of carbon dioxide gel for external use.

### Example 9

### Preparation of Granular Material (A)

A granular material (mean particle size: 0.3 mm) was prepared using 53.8 parts of sodium dihydrogenphosphate as the weak acid, 10.0 parts of disodium ethylenediamine tetraacetate as the calcium ion trapping agent, 5.0 parts of dextrin as the binder, 31.2 parts of lactose as the dispersant and a suitable amount of 50% ethanol.

### Preparation of Viscous Material (B)

A viscous material (B) was prepared the same way as in Example 6.
The resultant granular material (A) and viscous material (B) were paired to provide a composition for use in preparation of carbon dioxide gel for external use.

### Example 10

### Preparation of Granular Material (A)

A granular material (mean particle size: 0.3 mm) was prepared using 5.4 parts of sodium dihydrogenphosphate as the weak acid, 0.5 parts of disodium ethylenediamine tetraacetate as the calcium ion trapping agent, 5.0 parts of dextrin as the binder, 89.1 parts of lactose as the dispersant and a suitable amount of 50% ethanol.

### Preparation of Viscous Material (B)

A viscous material (B) was prepared using 0.4 parts of calcium carbonate, 3.5 parts of sodium alginate as the gelling agent gelated by calcium ions, 85.0 parts of purified water as the water, 0.9 parts of sodium carboxymethyl cellulose as the adhesive for increasing affinity to the skin/mucosa surface, 7.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 11

### Preparation of Granular Material (A)

A granular material (mean particle size: 0.3 mm) was prepared using 5.4 parts of sodium dihydrogenphosphate as the weak acid, 0.84 parts of disodium ethylenediamine tetraacetate as the calcium ion trapping agent, 5.0 parts of dextrin as the binder, 88.76 parts of lactose as the dispersant and a suitable amount of 50% ethanol.

### Preparation of Viscous Material (B)

A viscous material (B) was prepared the same way as in Example 10.
The resultant granular material (A) and viscous material (B) were paired to provide a composition for use in preparation of carbon dioxide gel for external use.

### Example 12

### Preparation of Granular Material (A)

A granular material (mean particle size: 0.3 mm) was prepared using 9.6 parts of sodium dihydrogenphosphate as the weak acid, 1.5 parts of disodium ethylenediamine tetraacetate as the calcium ion trapping agent, 5.0 parts of dextrin as the binding agent, 83.9 parts of lactose as the dispersant and a suitable amount of 50% ethanol.

### Preparation of Viscous Material (B)

A viscous material (B) was prepared the same way as in Example 10.
The resultant granular material (A) and viscous material (B) were paired to provide a composition for use in preparation of carbon dioxide gel for external use.

### Example 13

### Preparation of Granular Material (A)

A granular material (mean particle size: 0.3 mm) was prepared using 38.5 parts of sodium dihydrogenphosphate as the weak acid, 4.2 parts of disodium ethylenediamine tetraacetate as the calcium ion trapping agent, 5.0 parts of dextrin as the binder, 52.3 parts of lactose as the dispersant and a suitable amount of 50% ethanol.

### Preparation of Viscous Material (B)

A viscous material (B) was prepared the same way as in Example 10.
The resultant granular material (A) and viscous material (B) were paired to provide a composition for use in preparation of carbon dioxide gel for external use.

### Example 14 not exactly according to the invention

### Preparation of Granular Material (A)

A granular material (mean particle size: 0.3 mm) was prepared using 23.1 parts of sodium dihydrogenphosphate as the weak acid, 5.0 parts of disodium ethylenediamine tetraacetate as the calcium ion trapping agent and a suitable amount of 50% ethanol.

### Preparation of Viscous Material (B)

A viscous material (B) was prepared the same way as in Example 10.
The resultant granular material (A) and viscous material (B) were paired to provide a composition for use in preparation of carbon dioxide gel for external use.

### Example 15

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 5.0 parts of dextrin as the binder, 50.5 parts of lactose as the dispersant and a suitable amount of 50% ethanol.

### Preparation of Viscous Material (B)

A viscous material (B) was prepared the same way as in Example 10.
The resultant granular material (A) and viscous material (B) were paired to provide a composition for use in preparation of carbon dioxide topical gel for external use.

### Example 16

### Preparation of Granular Material (A)

A granular material (mean particle size: 0.3 mm) was prepared using 38.5 parts of sodium dihydrogenphosphate as the weak acid, 10.0 parts of disodium ethylenediamine tetraacetate as the calcium ion trapping agent, 5.0 parts of dextrin as the binder, 46.5 parts of lactose as the dispersant and a suitable amount of 50% ethanol.

### Preparation of Viscous Material (B)

A viscous material (B) was prepared the same way as in Example 10.
The resultant granular material (A) and viscous material (B) were paired to provide a composition for use in preparation of carbon dioxide gel for external use.

### Example 17

### Preparation of Granular Material (A)

A granular material (mean particle size: 0.3 mm) was prepared using 40.8 parts of sodium dihydrogenphosphate as the weak acid, 6.0 parts of disodium ethylenediamine tetraacetate as the calcium ion trapping agent, 5.0 parts of dextrin as the binder, 48.2 parts of lactose as the dispersant and a suitable amount of 50% ethanol.

### Preparation of Viscous Material (B)

A viscous material (B) was prepared the same way as in Example 10.
The resultant granular material (A) and viscous material (B) were paired to provide a composition for use in preparation of carbon dioxide gel for external use.

### Example 18

### Preparation of Granular Material (A)

A granular material (mean particle size: 0.3 mm) was prepared using 65.4 parts of sodium dihydrogenphosphate as the weak acid, 10.0 parts of disodium ethylenediamine tetraacetate as the calcium ion trapping agent, 5.0 parts of dextrin as the binder, 19.6 parts of lactose as the dispersant and a suitable amount of 50% ethanol.

### Preparation of Viscous Material (B)

A viscous material (B) was prepared the same way as in Example 10.
The resultant granular material (A) and viscous material (B) were paired to provide a composition for use in preparation of carbon dioxide gel for external use.

### Example 19

### Preparation of Granular Material (A)

A granular material (mean particle size: 0.3 mm) was prepared using 30.8 parts of sodium dihydrogenphosphate as the weak acid, 8.0 parts of disodium ethylenediamine tetraacetate as the calcium ion trapping agent, 5.0 parts of dextrin as the binder, 56.2 parts of lactose as the dispersant and a suitable amount of 50% ethanol.

### Preparation of Viscous Material (B)

A viscous material (B) was prepared using 0.5 parts of calcium carbonate, 3.5 parts of sodium alginate as the gelling agent gelated by calcium ions, 84.9 parts of purified water as the water, 0.9 parts of sodium carboxymethyl cellulose as the adhesive for increasing affinity to the skin/mucosa surface, 7.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 20

### Preparation of Granular Material (A)

A granular material (mean particle size: 0.3 mm) was prepared using 38.5 parts of sodium dihydrogenphosphate as the weak acid, 12 parts of disodium ethylenediamine tetraacetate as the calcium ion trapping agent, 5.0 parts of dextrin as the binder, 44.5 parts of lactose as the dispersant and a suitable amount of 50% ethanol.

### Preparation of Viscous Material (B)

A viscous material (B) was prepared the same way as in Example 19.
The resultant granular material (A) and viscous material (B) were paired to provide a composition for use in preparation of carbon dioxide gel for external use.

### Example 21

### Preparation of Granular Material (A)

A granular material (mean particle size: 0.3 mm) was prepared using 53.8 parts of sodium dihydrogenphosphate as the weak acid, 10.0 parts of disodium ethylenediamine tetraacetate as the calcium ion trapping agent, 5.0 parts of dextrin as the binding agent, 31.2 parts of lactose as the dispersant and a suitable amount of 50% ethanol.

### Preparation of Viscous Material (B)

A viscous material (B) was prepared the same way as in Example 19.
The resultant granular material (A) and viscous material (B) were paired to provide a composition for use in preparation of carbon dioxide gel for external use.

### Example 22

### Preparation of Granular Material (A)

A granular material (mean particle size: 0.3 mm) was prepared using 43.8 parts of sodium dihydrogenphosphate as the weak acid, 43.0 parts of disodium ethylenediamine tetraacetate as the calcium ion trapping agent, 13.2 parts of lactose as the dispersant and a suitable amount of 50% ethanol.

### Preparation of Viscous Material (B)

A viscous material (B) was prepared using 1.0 part of calcium carbonate, 3.5 parts of sodium alginate as the gelling agent gelated by calcium ions, 87.9 parts of purified water as the water, 0.9 parts of sodium carboxymethyl cellulose as the adhesive for increasing affinity to the skin/mucosa surface, 3.5 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 23

### Preparation of Granular Material (A)

A granular material (mean particle size: 0.3 mm) was prepared using 46.2 parts of sodium dihydrogenphosphate as the weak acid, 8.0 parts of disodium ethylenediamine tetraacetate as the calcium ion trapping agent, 5.0 parts of dextrin as the binder, 40.8 parts of lactose as the dispersant and a suitable amount of 50% ethanol.

### Preparation of Viscous Material (B)

A viscous material (B) was prepared the same way as in Example 22.
The resultant granular material (A) and viscous material (B) were paired to provide a composition for use in preparation of carbon dioxide gel for external use.

### Example 24

### Preparation of Granular Material (A)

A granular material (mean particle size: 0.3 mm) was prepared using 48.5 parts of sodium dihydrogenphosphate as the weak acid, 37.0 parts of disodium ethylenediamine tetraacetate as the calcium ion trapping agent, 14.5 parts of lactose as the dispersant and a suitable amount of 50% ethanol.

### Preparation of Viscous Material (B)

A viscous material (B) was prepared the same way as in Example 22.
The resultant granular material (A) and viscous material (B) were paired to provide a composition for use in preparation of carbon dioxide gel for external use.

### Example 25

### Preparation of Granular Material (A)

A granular material (mean particle size: 0.3 mm) was prepared using 50.0 parts of sodium dihydrogenphosphate as the weak acid, 35.0 parts of disodium ethylenediamine tetraacetate as the calcium ion trapping agent, 15.0 parts of lactose as the dispersant and a suitable amount of 50% ethanol.

### Preparation of Viscous Material (B)

A viscous material (B) was prepared the same way as in Example 22.
The resultant granular material (A) and viscous material (B) were paired to provide a composition for use in preparation of carbon dioxide gel for external use.

The following Examples 26 to 36 illustrate the first composition for use in preparation of carbon dioxide gel for external use. These examples use sodium dihydrogenphosphate or potassium dihydrogenphosphate as the weak acid of the granular material (A) and glycine as the calcium ion trapping agent of the granular material (A), as principally varying the mixing ratios thereof.

### Example 26

### Preparation of Granular Material (A)

A granular material (mean particle size: 0.3 mm) was prepared using 5.4 parts of sodium dihydrogenphosphate as the weak acid, 3.0 parts of glycine as the calcium ion trapping agent, 5.0 parts of dextrin as the binder, 86.6 parts of lactose as the dispersant and a suitable amount of 50% ethanol.

### Preparation of Viscous Material (B)

A viscous material (B) was prepared using 0.4 parts of calcium carbonate, 3.5 parts of sodium alginate as the gelling agent gelated by calcium ions, 85.0 parts of purified water as the water, 0.9 parts of sodium carboxymethyl cellulose as the adhesive for increasing affinity to the skin/mucosa surface, 7.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 27

### Preparation of Granular Material (A)

A granular material (mean particle size: 0.3 mm) was prepared using 5.4 parts of sodium dihydrogenphosphate as the weak acid, 5.6 parts of glycine as the calcium ion trapping agent, 5.0 parts of dextrin as the binder, 84.0 parts of lactose as the dispersant and a suitable amount of 50% ethanol.

### Preparation of Viscous Material (B)

A viscous material (B) was prepared the same way as in Example 26.
The resultant granular material (A) and viscous material (B) were paired to provide a composition for use in preparation of carbon dioxide gel for external use.

### Example 28

### Preparation of Granular Material (A)

A granular material (mean particle size: 0.3 mm) was prepared using 9.6 parts of potassium dihydrogenphosphate as the weak acid, 10.0 parts of glycine as the calcium ion trapping agent, 5.0 parts of dextrin as the binder, 75.4 parts of lactose as the dispersant and a suitable amount of 50% ethanol.

### Preparation of Viscous Material (B)

A viscous material (B) was prepared the same way as in Example 26.
The resultant granular material (A) and viscous material (B) were paired to provide a composition for use in preparation of carbon dioxide gel for external use.

### Example 29

### Preparation of Granular Material (A)

A granular material (mean particle size: 0.3 mm) was prepared using 28.5 parts of potassium dihydrogenphosphate as the weak acid, 21.2 parts of glycine as the calcium ion trapping agent, 5.0 parts of dextrin as the binder, 45.3 parts of lactose as the dispersant and a suitable amount of 50% ethanol.

### Preparation of Viscous Material (B)

A viscous material (B) was prepared the same way as in Example 26.
The resultant granular material (A) and viscous material (B) were paired to provide a composition for use in preparation of carbon dioxide gel for external use.

### Example 30

### Preparation of Granular Material (A)

A granular material (mean particle size: 0.3 mm) was prepared using 28.5 parts of sodium dihydrogenphosphate as the weak acid, 30.0 parts of glycine as the calcium ion trapping agent, 10.0 parts of dextrin as the binder, 31.5 parts of lactose as the dispersant and a suitable amount of 50% ethanol.

### Preparation of Viscous Material (B)

A viscous material (B) was prepared the same way as in Example 26.
The resultant granular material (A) and viscous material (B) were paired to provide a composition for use in preparation of carbon dioxide gel for external use.

### Example 31

### Preparation of Granular Material (A)

A granular material (mean particle size: 0.3 mm) was prepared using 28.5 parts of sodium dihydrogenphosphate as the weak acid, 49.4 parts of glycine as the calcium ion trapping agent, 5.0 parts of dextrin as the binder, 82.9 parts of lactose as the dispersant and a suitable amount of 50% ethanol.

### Preparation of Viscous Material (B)

A viscous material (B) was prepared the same way as in Example 26.
The resultant granular material (A) and viscous material (B) were paired to provide a composition for use in preparation of carbon dioxide gel for external use.

### Example 32 not exactly according to the invention

### Preparation of Granular Material (A)

A granular material (mean particle size: 0.3 mm) was prepared using 37.7 parts of sodium dihydrogenphosphate as the weak acid, 49.0 parts of glycine as the calcium ion trapping agent, 2.0 parts of dextrin as the binder and a suitable amount of 50% ethanol.

### Preparation of Viscous Material (B)

A viscous material (B) was prepared the same way as in Example 26.
The resultant granular material (A) and viscous material (B) were paired to provide a composition for use in preparation of carbon dioxide gel for external use.

### Example 33

### Preparation of Granular Material (A)

A granular material (mean particle size: 0.3m) was prepared using 38.5 parts of sodium dihydrogenphosphate as the weak acid, 12.0 parts of glycine as the calcium ion trapping agent, 5.0 parts of dextrin as the binder, 44.5 parts of lactose as the dispersant and a suitable amount of 50% ethanol.

### Preparation of Viscous Material (B)

A viscous material (B) was prepared the same way as in Example 26.
The resultant granular material (A) and viscous material (B) were paired to provide a composition for use in preparation of carbon dioxide gel for external use.

### Example 34 not exactly according to the invention

### Preparation of Granular Material (A)

A granular material (mean particle size: 0.3 mm) was prepared using 40.5 parts of sodium dihydrogenphosphate as the weak acid, 42.3 parts of glycine as the calcium ion trapping agent and a suitable amount of 50% ethanol.

### Preparation of Viscous Material (B)

A viscous material (B) was prepared the same way as in Example 26.
The resultant granular material (A) and viscous material (B) were paired to provide a composition for use in preparation of carbon dioxide gel for external use.

### Example 35 not exactly according to the invention

### Preparation of Granular Material (A)

A granular material (mean particle size: 0.3 mm) was prepared using 65.4 parts of sodium dihydrogenphosphate as the weak acid, 15.0 parts of glycine as the calcium ion trapping agent and a suitable amount of 50% ethanol.

### Preparation of Viscous Material (B)

A viscous material (B) was prepared the same way as in Example 26.
The resultant granular material (A) and viscous material (B) were paired to provide a composition for use in preparation of carbon dioxide gel for external use.

### Example 36 not exactly according to the invention

### Preparation of Granular Material (A)

A granular material (mean particle size: 0.3 mm) was prepared using 74.6 parts of sodium dihydrogenphosphate as the weak acid, 3.0 parts of glycine as the calcium ion trapping agent and a suitable amount of 50% ethanol.

### Preparation of Viscous Material (B)

A viscous material (B) was prepared the same way as in Example 26.
The resultant granular material (A) and viscous material (B) were paired to provide a composition for use in preparation of carbon dioxide gel for external use.

The following Examples 37 to 40 illustrate the second composition for use in preparation of carbon dioxide gel for external use. These examples use disodium hydrogenphosphate as the calcium ion trapping agent of the viscous material (Y) and sodium dihydrogenphosphate as the weak acid of the granular material (X), as varying the mixing ratio of sodium dihydrogenphosphate.

### Example 37

### Preparation of Granular Material (X)

A granular material (mean particle size: 0.3 mm) was prepared using 26.9 parts of sodium dihydrogenphosphate as the weak acid, 5.0 parts of dextrin as the binder, 68.1 parts of lactose as the dispersant and a suitable amount of 50% ethanol.

### Preparation of Viscous Material (Y)

A viscous material (Y) was prepared using 0.4 parts of calcium carbonate, 3.5 parts of sodium alginate as the gelling agent gelated by calcium ions, 0.7 parts of disodium hydrogenphosphate dihydrate as the calcium ion trapping agent, 84.3 parts of purified water as the water, 0.9 parts of sodium carboxymethyl cellulose as the adhesive for increasing affinity to the skin/mucosa surface, 7.0 parts of 1,3-butylene glycol as the alcohol, 0.5 parts of phenoxyethanol as the preservative and 2.7 parts of pentylene glycol as the alcohol and preservative.
The resultant granular material (X) and viscous material (Y) were paired to provide a composition for use in preparation of carbon dioxide gel for external use.

### Example 38

### Preparation of Granular Material (X)

A granular material (mean particle size: 0.3 mm) was prepared using 34.6 parts of sodium dihydrogenphosphate as the weak acid, 5.0 parts of dextrin as the binder, 60.4 parts of lactose as the dispersant and a suitable amount of 50% ethanol.

### Preparation of Viscous Material (Y)

A viscous material (Y) was prepared the same way as in Example 37.
The resultant granular material (X) and viscous material (Y) were paired to provide a composition for use in preparation of carbon dioxide gel for external use.

### Example 39

### Preparation of Granular Material (X)

A granular material (mean particle size: 0.3 mm) was prepared using 42.3 parts of sodium dihydrogenphosphate as the weak acid, 5.0 parts of dextrin as the binder, 52.7 parts of lactose as the dispersant and a suitable amount of 50% ethanol.

### Preparation of Viscous Material (Y)

A viscous material (Y) was prepared the same way as in Example 37.
The resultant granular material (X) and viscous material (Y) were paired to provide a composition for use in preparation of carbon dioxide gel for external use.

### Example 40

### Preparation of Granular Material (X)

A granular material (mean particle size: 0.3 mm) was prepared using 50.0 parts of sodium dihydrogenphosphate as the weak acid, 5.0 parts of dextrin as the binder, 45.0 parts of lactose as the dispersant and a suitable amount of 50% ethanol.

### Preparation of Viscous Material (Y)

A viscous material (Y) was prepared the same way as in Example 37.
The resultant granular material (X) and viscous material (Y) were paired to provide a composition for use in preparation of carbon dioxide gel for external use.

The following Examples 41 to 59 illustrate the first composition for use in preparation of carbon dioxide gel for external use. In these examples, the granular material (A) is admixed with the binder, the type and mixing ratio of which are varied.

### Example 41

### Preparation of Granular Material (A)

A granular material (mean particle size: 0.3 mm) was prepared using 40.8 parts of sodium dihydrogenphosphate as the weak acid, 6.0 parts of disodium ethylenediamine tetraacetate as the calcium ion trapping agent, 5.0 parts of dextrin as the binder, 10.0 parts of potato starch as the binder, 38.2 parts of lactose as the dispersant and a suitable amount of 50% ethanol.

### Preparation of Viscous Material (B)

A viscous material (B) was prepared using 0.4 parts of calcium carbonate, 3.5 parts of sodium alginate as the gelling agent gelated by calcium ions, 85.0 parts of purified water as the water, 0.9 parts of sodium carboxymethyl cellulose as the adhesive for increasing affinity to the skin/mucosa surface, 7.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 42

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 5.0 parts of dextrin as the binder, 10.0 parts of potato starch as the binder, 40.5 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

### Example 43

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 5.0 parts of sodium carboxymethyl cellulose as the binder, 50.5 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

### Example 44

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 5.0 parts of methyl cellulose as the binder, 50.5 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

### Example 45

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 5.0 parts of hydroxypropyl starch as the binder, 50.5 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

### Example 46

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 5.0 parts of dextrin and 5.0 parts of hydroxypropyl starch as the binder, 45.5 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use

### Example 47

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 5.0 parts of potato starch as the bindernt, 50.5 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

### Example 48

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 5.0 parts of hydroxypropyl cellulose as the binder, 50.5 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

### Example 49

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 5.0 parts of hydroxypropyl cellulose and 10.0 parts of potato starch as the binder, 40.5 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

### Example 50

### Preparation of Granular Material (A)

A granular material (mean particle size: 0.3 mm) was prepared using 50.0 parts of sodium dihydrogenphosphate as the weak acid, 6.0 parts of disodium ethylenediamine tetraacetate as the calcium ion trapping agent, 5.0 parts of hydroxypropyl cellulose as the binder, 39.0 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

### Example 51

### Preparation of Granular Material (A)

A granular material (mean particle size: 0.3 mm) was prepared using 50.0 parts of sodium dihydrogenphosphate as the weak acid, 6.0 parts of disodium ethylenediamine tetraacetate as the calcium ion trapping agent, 5.0 parts of hydroxypropyl cellulose as the binder, 39.0 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

### Example 52

### Preparation of Granular Material (A)

A granular material (mean particle size: 0.3 mm) was prepared using 50.0 parts of sodium dihydrogenphosphate as the weak acid, 6.0 parts of disodium ethylenediamine tetraacetate as the calcium ion trapping agent, 5.0 parts of hydroxypropyl cellulose and 10.0 parts of potato starch as the binder, 29.0 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

### Example 53

### Preparation of Granular Material (A)

A granular material (mean particle size: 0.3 mm) was prepared using 50.0 parts of sodium dihydrogenphosphate as the weak acid, 6.0 parts of disodium ethylenediamine tetraacetate as the calcium ion trapping agent, 5.0 parts of hydroxypropylmethyl cellulose as the binder, 39.0 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

### Example 54

### Preparation of Granular Material (A)

A granular material (mean particle size: 0.3 mm) was prepared using 50.0 parts of sodium dihydrogenphosphate as the weak acid, 6.0 parts of disodium ethylenediamine tetraacetate as the calcium ion trapping agent, 5.0 parts of hydroxypropylmethyl cellulose as the binder, 39.0 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

### Example 55

### Preparation of Granular Material (A)

A granular material (mean particle size: 0.3 mm) was prepared using 50.0 parts of sodium dihydrogenphosphate as the weak acid, 6.0 parts of disodium ethylenediamine tetraacetate as the calcium ion trapping agent, 5.0 parts of hydroxypropylmethyl cellulose as the binder, 39.0 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

### Example 56

### Preparation of Granular Material (A)

A granular material (mean particle size: 0.3 mm) was prepared using 50.0 parts of sodium dihydrogenphosphate as the weak acid, 6.0 parts of disodium ethylenediamine tetraacetate as the calcium ion trapping agent, 10.0 parts of dextrin as the binder, 34.0 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

### Example 57

### Preparation of Granular Material (A)

A granular material (mean particle size: 0.3 mm) was prepared using 50.0 parts of sodium dihydrogenphosphate as the weak acid, 6.0 parts of disodium ethylenediamine tetraacetate as the calcium ion trapping agent, 10.0 parts of dextrin and 5.0 parts of low substituted hydroxypropylcellulose as the binder, 29.0 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

### Example 58

### Preparation of Granular Material (A)

A granular material (mean particle size: 0.3 mm) was prepared using 50.0 parts of sodium dihydrogenphosphate as the weak acid, 6.0 parts of disodium ethylenediamine tetraacetate as the calcium ion trapping agent, 10.0 parts of dextrin and 2.0 parts of low susbstituted hydroxypropylcellulose as the binder, 32.0 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

### Example 59

### Preparation of Granular Material (A)

A granular material (mean particle size: 0.3 mm) was prepared using 50.0 parts of sodium dihydrogenphosphate as the weak acid, 6.0 parts of disodium ethylenediamine tetraacetate as the calcium ion trapping agent, 10.0 parts of dextrin and 5.0 parts of crystalline cellulose as the binder, 29.0 parts of lactose as the dispersant and a suitable amount of 50% ethanol.

### Preparation of Viscous Material (B)

A viscous material (B) was prepared the same way as in Example 41.
The resultant granular material (A) and viscous material (B) were paired to provide a composition for use in preparation of carbon dioxide gel for external use.

The following Examples 60 to 69 illustrate the first composition for use in preparation of carbon dioxide gel for external use. In these examples, the granular material (A) is admixed with the dispersant, the type of which is varied.

### Example 60

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 10.0 parts of dextrin as the binder, 45.5 parts of lactose as the dispersant and a suitable amount of 50% ethanol.

### Preparation of Viscous Material (B)

A viscous material (B) was prepared using 0.4 parts of calcium carbonate, 3.5 parts of sodium alginate as the gelling agent gelated by calcium ions, 85.0 parts of purified water as the water, 0.9 parts of sodium carboxymethyl cellulose as the adhesive for increasing affinity to the skin/mucosa surface, 7.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 61

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 10.0 parts of dextrin as the binder, 45.5 parts of glucose as the dispersant and a suitable amount of 50% ethanol.

### Preparation of Viscous Material (B)

A viscous material (B) was prepared the same way as in Example 60.
The resultant granular material (A) and viscous material (B) were paired to provide a composition for use in preparation of carbon dioxide gel for external use.

### Example 62

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 10.0 parts of dextrin as the binder, 45.5 parts of saccharose as the dispersant and a suitable amount of 50% ethanol.

### Preparation of Viscous Material (B)

A viscous material (B) was prepared the same way as in Example 60.
The resultant granular material (A) and viscous material (B) were paired to provide a composition for use in preparation of carbon dioxide gel for external use.

### Example 63

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 10.0 parts of dextrin as the binder, 45.5 parts of xylitol as the dispersant and a suitable amount of 50% ethanol.

### Preparation of Viscous Material (B)

A viscous material (B) was prepared the same way as in Example 60.
The resultant granular material (A) and viscous material (B) were paired to provide a composition for use in preparation of carbon dioxide gel for external use.

### Example 64

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 10.0 parts of dextrin as the binder, 45.5 parts of sorbitol as the dispersant and a suitable amount of 50% ethanol.

### Preparation of Viscous Material (B)

A viscous material (B) was prepared the same way as in Example 60.
The resultant granular material (A) and viscous material (B) were paired to provide a composition for use in preparation of carbon dioxide gel for external use.

### Example 65

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 10.0 parts of dextrin as the binder, 45.5 parts of mancitol as the dispersant and a suitable amount of 50% ethanol.

### Preparation of Viscous Material (B)

A viscous material (B) was prepared the same way as in Example 60.
The resultant granular material (A) and viscous material (B) were paired to provide a composition for use in preparation of carbon dioxide gel for external use.

### Example 66

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 10.0 parts of dextrin as the binder, 45.5 parts of mannitol as the dispersant and a suitable amount of 50% ethanol.

### Preparation of Viscous Material (B)

A viscous material (B) was prepared the same way as in Example 60.
The resultant granular material (A) and viscous material (B) were paired to provide a composition for use in preparation of carbon dioxide gel for external use.

### Example 67

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 10.0 parts of dextrin as the binder, 45.5 parts of urea as the dispersant and a suitable amount of 50% ethanol.

### Preparation of Viscous Material (B)

A viscous material (B) was prepared the same way as in Example 60.
The resultant granular material (A) and viscous material (B) were paired to provide a composition for use in preparation of carbon dioxide gel for external use.

### Example 68

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 10.0 parts of dextrin as the binder, 45.5 parts of xanthan gum as the dispersant and a suitable amount of 50% ethanol.

### Preparation of Viscous Material (B)

A viscous material (B) was prepared the same way as in Example 60.
The resultant granular material (A) and viscous material (B) were paired to provide a composition for use in preparation of carbon dioxide gel for external use.

### Example 69

### Preparation of Granular Material (A)

A granular material (mean particle size: 0.3 mm) was prepared using 38.5 parts of sodium dihydrogenphosphate as the weak acid, 6.0 parts of disodium ethylenediamine tetraacetate as the calcium ion trapping agent, 10.0 parts of dextrin as the binder, 45.5 parts of pullulan as the dispersant and a suitable amount of 50% ethanol.

### Preparation of Viscous Material (B)

A viscous material (B) was prepared the same way as in Example 60.
The resultant granular material (A) and viscous material (B) were paired to provide a composition for use in preparation of carbon dioxide gel for external use.

The following Examples 70 to 86 illustrate the first composition for use in preparation of carbon dioxide gel for external use. In these examples, the viscous material (B) is admixed with the adhesive and the like for increasing affinity to the skin/mucosa surface, and is varied in the formulation.

### Example 70

### Preparation of Granular Material (A)

A granular material (mean particle size: 0.3 mm) was prepared using 53.0 parts of sodium dihydrogenphosphate as the weak acid, 6.0 parts of disodium ethylenediamine tetraacetate as the calcium ion trapping agent, 5.0 parts of dextrin as the binder, 34.0 parts of lactose as the dispersant and a suitable amount of 50% ethanol.

### Preparation of Viscous Material (B)

A viscous material (B) was prepared using 0.4 parts of calcium carbonate, 3.5 parts of sodium alginate as the gelling agent gelated by calcium ions, 79.0 parts of purified water as the water, 0.9 parts of sodium carboxymethyl cellulose as the adhesive for increasing affinity to the skin/mucosa surface, 10.0 parts of 1,3-butylene glycol as the alcohol, 0.5 parts of phenoxyethanol as the preservative and 5.7 parts of pentylene glycol as the alcohol and preservative.
The resultant granular material (A) and viscous material (B) were paired to provide a composition for use in preparation of carbon dioxide gel for external use.

### Example 71

### Preparation of Granular Material (A)

A granular material (A) was prepared the same way as in Example 70.

### Preparation of Viscous Material (B)

A viscous material (B) was prepared using 0.4 parts of calcium carbonate, 3.5 parts of sodium alginate as the gelling agent gelated by calcium ions, 82.0 parts of purified water as the water, 0.9 parts of sodium carboxymethyl cellulose as the adhesive for increasing affinity to the skin/mucosa surface, 10.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 72

### Preparation of Granular Material (A)

A granular material (A) was prepared the same way as in Example 70.

### Preparation of Viscous Material (B)

A viscous material (B) was prepared using 0.4 parts of calcium carbonate, 3.5 parts of sodium alginate as the gelling agent gelated by calcium ions, 84.7 parts of purified water as the water, 0.9 parts of sodium carboxymethyl cellulose as the adhesive for increasing affinity to the skin/mucosa surface, 10.0 parts of 1,3-butylene glycol as the alcohol and 0.5 parts of phenoxyethanol as the preservative.
The resultant granular material (A) and viscous material (B) were paired to provide a composition for use in preparation of carbon dioxide gel for external use.

### Example 73

### Preparation of Granular Material (A)

A granular material (A) was prepared the same way as in Example 70.

### Preparation of Viscous Material (B)

A viscous material (B) was prepared using 0.4 parts of calcium carbonate, 3.5 parts of sodium alginate as the gelling agent gelated by calcium ions, 82.0 parts of purified water as the water, 0.9 parts of sodium carboxymethyl cellulose as the adhesive for increasing affinity to the skin/mucosa surface, 7.0 parts of 1,3-butylene glycol as the alcohol, 0.5 parts of phenoxyethanol as the preservative and 5.7 parts of pentylene glycol as the alcohol and preservative.
The resultant granular material (A) and viscous material (B) were paired to provide a composition for use in preparation of carbon dioxide gel for external use.

### Example 74

### Preparation of Granular Material (A)

A granular material (A) was prepared the same way as in Example 70.

### Preparation of Viscous Material (B)

A viscous material (B) was prepared using 0.4 parts of calcium carbonate, 3.5 parts of sodium alginate as the gelling agent gelated by calcium ions, 84.4 parts of purified water as the water, 7.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 75

### Preparation of Granular Material (A)

A granular material (A) was prepared the same way as in Example 70.

### Preparation of Viscous Material (B)

A viscous material (B) was prepared using 0.4 parts of calcium carbonate, 4.0 parts of sodium alginate as the gelling agent gelated by calcium ions, 84.9 parts of purified water as the water, 0.5 parts of sodium carboxymethyl cellulose as the adhesive for increasing affinity to the skin/mucosa surface, 7.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 76

### Preparation of Granular Material (A)

A granular material (A) was prepared the same way as in Example 70.

### Preparation of Viscous Material (B)

A viscous material (B) was prepared using 0.4 parts of calcium carbonate, 3.5 parts of sodium alginate as the gelling agent gelated by calcium ions, 84.4 parts of purified water as the water, 1.5 parts of sodium carboxymethyl cellulose as the adhesive for increasing affinity to the skin/mucosa surface, 7.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 77

### Preparation of Granular Material (A)

A granular material (A) was prepared the same way as in Example 70.

### Preparation of Viscous Material (B)

A viscous material (B) was prepared using 0.4 parts of calcium carbonate, 3.5 parts of sodium alginate as the gelling agent gelated by calcium ions, 86.0 parts of purified water as the water, 0.9 parts of sodium carboxymethyl cellulose as the adhesive for increasing affinity to the skin/mucosa surface, 6.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 78

### Preparation of Granular Material (A)

A granular material (A) was prepared the same way as in Example 70.

### Preparation of Viscous Material (B)

A viscous material (B) was prepared using 0.4 parts of calcium carbonate, 2.9 parts of sodium alginate as the gelling agent gelated by calcium ions, 85.0 parts of purified water as the water, 1.5 parts of sodium carboxymethyl cellulose as the adhesive for increasing affinity to the skin/mucosa surface, 7.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 79

### Preparation of Granular Material (A)

A granular material (A) was prepared the same way as in Example 70.

### Preparation of Viscous Material (B)

A viscous material (B) was prepared using 0.4 parts of calcium carbonate, 1.9 parts of sodium alginate as the gelling agent gelated by calcium ions, 85.0 parts of purified water as the water, 2.5 parts of sodium carboxymethyl cellulose as the adhesive for increasing affinity to the skin/mucosa surface, 7.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 80

### Preparation of Granular Material (A)

A granular material (A) was prepared the same way as in Example 70.

### Preparation of Viscous Material (B)

A viscous material (B) was prepared using 0.4 parts of calcium carbonate, 0.5 parts of sodium alginate as the gelling agent gelated by calcium ions, 85.0 parts of purified water as the water, 3.9 parts of sodium carboxymethyl cellulose as the adhesive for increasing affinity to the skin/mucosa surface, 7.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 81

### Preparation of Granular Material (A)

A granular material (A) was prepared the same way as in Example 70.

### Preparation of Viscous Material (B)

A viscous material (B) was prepared using 0.4 parts of calcium carbonate, 3.5 parts of sodium alginate as the gelling agent gelated by calcium ions, 88.7 parts of purified water as the water, 0.9 parts of sodium carboxymethyl cellulose as the adhesive for increasing affinity to the skin/mucosa surface, 6.0 parts of 1,3-butylene glycol as the alcohol and 0.5 parts of phenoxyethanol as the preservative.
The resultant granular material (A) and viscous material (B) were paired to provide a composition for use in preparation of carbon dioxide gel for external use.

### Example 82

### Preparation of Granular Material (A)

A granular material (A) was prepared the same way as in Example 70.

### Preparation of Viscous Material (B)

A viscous material (B) was prepared using 0.4 parts of calcium carbonate, 3.5 parts of sodium alginate as the gelling agent gelated by calcium ions, 88.0 parts of purified water as the water, 0.9 parts of sodium carboxymethyl cellulose as the adhesive for increasing affinity to the skin/mucosa surface, 4.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 83

### Preparation of Granular Material (A)

A granular material (A) was prepared the same way as in Example 70.

### Preparation of Viscous Material (B)

A viscous material (B) was prepared using 0.4 parts of calcium carbonate, 3.5 parts of sodium alginate as the gelling agent gelated by calcium ions, 89.0 parts of purified water as the water, 0.9 parts of sodium carboxymethyl cellulose as the adhesive for increasing affinity to the skin/mucosa surface, 0.5 parts of phenoxyethanol as the preservative and 5.7 parts of pentylene glycol as the alcohol and preservative.
The resultant granular material (A) and viscous material (B) were paired to provide a composition for use in preparation of carbon dioxide gel for external use.

### Example 84

### Preparation of Granular Material (A)

A granular material (A) was prepared the same way as in Example 70.

### Preparation of Viscous Material (B)

A viscous material (B) was prepared using 0.4 parts of calcium carbonate, 3.5 parts of sodium alginate as the gelling agent gelated by calcium ions, 92.0 parts of purified water as the water, 0.9 parts of sodium carboxymethyl cellulose as the adhesive for increasing affinity to the skin/mucosa surface, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 85

### Preparation of Granular Material (A)

A granular material (A) was prepared the same way as in Example 70.

### Preparation of Viscous Material (B)

A viscous material (B) was prepared using 0.4 parts of calcium carbonate, 3.5 parts of sodium alginate as the gelling agent gelated by calcium ions, 94.7 parts of purified water as the water, 0.9 parts of sodium carboxymethyl cellulose as the adhesive for increasing affinity to the skin/mucosa surface and 0.5 parts of phenoxyethanol as the preservative.
The resultant granular material (A) and viscous material (B) were paired to provide a composition for use in preparation of carbon dioxide gel for external use.

### Example 86

### Preparation of Granular Material (A)

A granular material (A) was prepared the same way as in Example 70.

### Preparation of Viscous Material (B)

A viscous material (B) was prepared using 0.3 parts of calcium carbonate, 3.5 parts of sodium alginate as the gelling agent gelated by calcium ions, 85.1 parts of purified water as the water, 0.9 parts of sodium carboxymethyl cellulose as the adhesive for increasing affinity to the skin/mucosa surface, 7.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 87

### Preparation of Granular Material (A)

A granular material (mean particle size: 0.3 mm) was prepared using 5.4 parts of sodium dihydrogenphosphate as the weak acid, 0.5 parts of disodium ethylenediamine tetraacetate as the calcium ion trapping agent, 5.0 parts of dextrin as the binder, 89.1 parts of lactose as the dispersant and a suitable amount of 50% ethanol.

### Preparation of Viscous Material (B)

A viscous material (B) was prepared using 6.0 parts of calcium carbonate, 3.5 parts of sodium alginate as the gelling agent gelated by calcium ions, 79.4 parts of purified water as the water, 0.9 parts of sodium carboxymethyl cellulose as the adhesive for increasing affinity to the skin/mucosa surface, 7.0 parts of 1,3-butylene glycol as the alcohol, 0.5 parts of phenoxyethanol as the preservative and 2.7 parts of pentylene glycol as the alcohol and preservative.
The resultant granular material (A) and viscous material (B) were paired to provide a composition for use in preparation of carbon dioxide gel for external use.

### Example 88

### Preparation of Granular Material (A)

A granular material (mean particle size: 0.3 mm) was prepared using 38.5 parts of sodium dihydrogenphosphate as the weak acid, 4.2 parts of disodium ethylenediamine tetraacetate as the calcium ion trapping agent, 5.0 parts of dextrin as the binder, 52.3 parts of lactose as the dispersant and a suitable amount of 50% ethanol.

### Preparation of Viscous Material (B)

A viscous material (B) was prepared using 2.7 parts of calcium carbonate, 14.6 parts of sodium alginate as the gelling agent gelated by calcium ions and 82.7 parts of purified water as the water.
The resultant granular material (A) and viscous material (B) were paired to provide a composition for use in preparation of carbon dioxide gel for external use.

### Comparative Example 1

The following materials were prepared as follows according to Example 109 in Japanese Patent Application Laid-open No. 2000-319187.

### Preparation of Granular Materials

Porous columnar granules having a length of about 4 mm and a diameter of about 1mm were prepared by wet extrusion granulation using water as a solvent, wherein 25 parts of citric acid, 25 parts of ethyl cellulose and 50 parts of croscarmellose sodium were used.

### Preparation of Viscous Composition

2.4 parts of sodium hydrogen carbonate was dissolved in 89.6 parts of purified water. With slow heating to 60 °C, 4.0 parts of sodium alginate, 2.0 parts of ethyl cellulose and 2.0 parts of sodium carboxymethyl cellulose were slowly added to the resultant solution mixture and were dissolved therein by stirring. The resultant solution was allowed to stand overnight and was cooled to the room temperature. Thus was obtained a viscous composition.
The resultant granules and viscous composition were paired to provide a composition for use in preparation of carbon dioxide agent for external use.

### Comparative Example 2

The following materials were prepared as follows according to Example 1 in Publication No. WO 02/08941.

### Preparation of Granular Materials

Porous columnar granules having a length of about 4 mm and a diameter of about lmm were prepared by wet extrusion granulation using water as a solvent, wherein 50 parts of lactose as a water-soluble dispersant, 30 parts of citric acid as a water-soluble acid and 7 parts of processed starch, 3 parts of dextrin and 10 parts of potato starch as thickeners.

### Preparation of Viscous Composition

4.0 parts of sodium hydrogen carbonate as a carbonate was dissolved in 91.5 parts of purified water. With slow heating to 60 °C, 1.5 parts of sodium alginate and 3.0 parts of sodium carboxymethyl cellulose as thickeners were slowly added to the resultant solution mixture and were dissolved therein by stirring. The resultant solution was allowed to stand overnight and was cooled to the room temperature. Thus was obtained a viscous composition.
The resultant granules and viscous composition were paired to provide a composition for use in preparation of carbon dioxide agent for external use.

The compositions for use in preparation of carbon dioxide gel for external use of the examples and the compositions for use in preparation of carbon dioxide topical agent of the comparative examples were evaluated as follows.

### Evaluation 1: Partial Face Slimming, Whitening and Skin Smoothening Effects

The compositions for use in preparation of carbon dioxide gel for external use of Examples 1 to 5 were each used as follows to prepare a carbon dioxide gel for external use. That is, 1.2 g of granular material and 20 g of viscous material were manually mixed with a plastic spatula which was moved round and round 30 times.
The composition for use in preparation of carbon dioxide agent for external use of Comparative Example 1 was used as follows to prepare a carbon dioxide agent for external use. That is, 2 g of granular material and 30 g of viscous material were manually mixed with a plastic spatula which was moved round and round 30 times.
The composition for use in preparation of carbon dioxide agent for external use of Comparative Example 2 was used as follows to prepare a carbon dioxide agent for external use. That is, 1.4 g of granular material and 30 g of viscous material were manually mixed with a plastic spatula which was turned round and round 30 times.

All the compositions for use in preparation of carbon dioxide gel for external use of Examples 1 to 5 offered easy mixing of the granular material and the viscous material. All the compositions of the examples formed the gels in which the granular material was homogeneously dispersed and dissolved in the viscous material and in which practically no carbon dioxide bubbles were observed. There were obtained the liquid gels having proper viscosity, adhesiveness and softness.
The resultant liquid gels were applied to the faces of female subjects aged 27 to 42 in a thickness of about 0.5 mm, respectively. Each of the gels exhibited sufficient fluidity and good spreadability, so as to be applied to the overall face surface in a uniform thickness. Any of the gels did not dropped down from the face. Immediately after the application, all the gels delivered an extremely notable cooling sensation which was sustained while the gels were left on the face. Since the generation of carbon dioxide is based on an endothermic reaction between the acid and carbonate, the sustention of cooling sensation of the gel indicates that the reaction is sustained. With all the gels, the subjects felt their facial skin toned or shrunk. The gels were removed 15 minutes after the application. The gels prepared from the compositions for use in preparation of carbon dioxide gel for external use of Examples 1 to 5 were all adequately solidified to form hydrogel sheets, which were readily peeled off from the face surfaces. All the subjects who used the gels reported that the gels made their skin appear more white, smoother and clearer and lightened the darkened areas as compared with their skin prior to the application of the gels. Furthermore, all the gels achieved a partial slimming effect to slim cheeks and a lift-up effect to lift up the cheeks and the angle of mouth. Particularly, the gel prepared from the composition for use in preparation of carbon dioxide gel for external use of Example 2 was superior in both the application feeling such as ease of application and ease of peeling-off, and the efficacy. As left on the skin for more than 30 minutes, all the gels sustained the cooling sensation and achieved the aforesaid aesthetic effect.

On the other hand, the composition for use in preparation of carbon dioxide agent for external use of Comparative Example 1 provided the easy mixing of the granular material and the viscous material but the resultant gel contained numerous bubbles, which were broken when pressed with a spatula. When the resultant carbon dioxide agent for external use was applied to subjects' cheeks, the agent immediately dropped down from the cheeks to soil the subjects' cloth. Although the agent delivered the cooling sensation immediately after the application, the effect was not sustained for more than three minutes. This revealed that the carbon dioxide generation reaction is not sustainable. The above carbon dioxide agent for external use was removed 15 minutes after the application. However, the agent had such a high viscosity that the agent was not removed simply by washing with water and required washing with soap. After the removal of the agent, the skin appeared slightly whitened. However, the agent did not achieve the partial slimming effect to slim the cheeks nor the lift-up effect to lift up the cheeks and the angle of mouth.

In the case of the carbon dioxide gel for external use prepared from the composition for use in preparation of carbon dioxide gel for external use according to the present invention, about 20 g of the gel was more than enough to cover the overall face area. In the case of the carbon dioxide agent for external use prepared from the composition for use in preparation of carbon dioxide agent for external use of Comparative Example 1, however, about 30 g of the agent was slightly insufficient for covering the overall face area as applied to the face in manner not to break the bubbles in the above carbon dioxide agent for external use.
On the other hand, the composition for use in carbon dioxide agent for external use of Comparative Example 2 provided the easy mixing of the granular material and the viscous material, permitting the granular material to be homogeneously dispersed and dissolved in the viscous material. Furthermore, a sufficient amount of carbon dioxide was generated. When the resultant carbon dioxide agent for external use was applied to subjects' cheeks, the agent did not drop down from the cheeks. The agent was removed 15 minutes after the application and the subjects' cheeks were observed. Unfortunately, the agent delivered lower effects, such as whitening, than the gels of Examples 1 to 5. Furthermore, the subjects must wash their faces with water in order to completely remove the carbon dioxide agent for external use applied to the cheeks. Hence, some of the subjects complained it was a little bit bothersome.

### Evaluation 2: Partial Arm Slimming, Whitening and Skin Smoothening Effects

The composition for use in preparation of carbon dioxide gel for external use of Example 2 was used as follows to prepare a carbon dioxide gel for external use. That is, 4 g of granular material (A) and 60 g of viscous material (B) were manually mixed with a plastic spatula which was moved round and round 30 times. The composition formed a gel in which the granular material was homogeneously dispersed and dissolved in the viscous material and in which practically no carbon dioxide bubbles were observed. Thus was obtained the liquid gel having proper viscosity, adhesiveness and softness. When the resultant liquid gel was applied to a right upper arm of a 35-year-old woman, the gel exhibited such good fluidity and spreadability as to be applied in a uniform thickness without dropping down. Immediately after the application, the liquid gel delivered an extremely notable cooling sensation which was sustained while the gel was left on the arm. The gel was removed 25 minutes after the application. The gel was adequately solidified to form a hydrogel sheet, which was readily peeled off from the upper arm. Measurement was taken on the circumference of the upper arm at a longitudinal mid point. A post-application measurement was 26.5 cm, which was 1 cm decreased from a pre-application measurement, 27.5 cm. Thus, the gel achieved the partial slimming effect. As compared with a surrounding area of the application area, the gel application area obviously appeared whitened and smoothened.

### Evaluation 3: Wound Treatment (1)

A carbon dioxide gel for external use was prepared using 0.5 g of the granular material (A) and 7 g of the viscous material (B) of the composition for use in preparation of carbon dioxide gel for external use of Example 1. The resultant gel was applied to an abrasion (1 cm × 3 cm) on the left elbow of a 9-year-old girl and was left on the area for 30 minutes. Immediately after the preparation, the gel was soft and highly spreadable and hence, was easy to apply. The gel continued to deliver the cooling sensation to the applied area while the gel was left on the area. Thus, the pain and itchy sensation associated with the abrasion was eliminated. The gel was removed 30 minutes after the application. The gel was adequately solidified to form a hydrogel sheet, which was readily peeled off from the applied area. The treatment procedure was conducted once a day and continued for five days. The wound was completely closed and healed with no skin pigmentation observed.

### Evaluation 4: Treatment of Contact Dermatitis

In order to treat contact dermatitis with rush (4 cm × 7 cm area on the left side of the forehead) of 27-year-old woman, a carbon dioxide gel for external use was prepared using 0.5 g of the granular material (A) and 15 g of the viscous material (B) of the composition for use in preparation of carbon dioxide gel for external use of Example 4. The resultant gel was applied to the affected area and was left on the area for 20 minutes. Immediately after the preparation, the gel was soft and highly spreadable and hence, was easy to apply. The gel continued to deliver the cooling sensation to the applied area while the gel was left on the area. Thus, the itchy sensation accompanying the dermatitis was eliminated. The gel was removed 20 minutes after the application. The gel was adequately solidified to form a hydrogel sheet, which was readily peeled off from the applied area. After the removal of the gel, the affected area was cured with no skin rush and roughness observed.

### Evaluation 5: Reaction Caused by Generation and Absorption of Carbon Dioxide

The following evaluation tests were all conducted on a 27-year-old female subject.
Each of the compositions for use in preparation of carbon dioxide gel for external use of Examples 6 to 9 was used as follows to prepare a carbon dioxide gel for external use. That is, the gel for external use was prepared using 0.2 g of the granular material (A) and 3.0 g of the viscous material (B) of each of the compositions. The resultant gels were each applied to the forearm or the forefoot. All the applied gels caused reactions (skin reddening, cooling sensation and such) which were effected by absorbing the generated carbon dioxide. The applied carbon dioxide gels for external use were not solidified within five minutes after the preparation and could be spread on the skin. The applied gels for external use were solidified at their surfaces in 15 to 20 minutes. After the lapse of 30 minutes from the application, the gels for external use formed hydrogel sheets, respectively. Thereafter, the individual hydrogel sheets were completely peeled off from the skin and quite favorable results were achieved.

Each of the compositions for use in preparation of carbon dioxide gel for external use of Examples 10 to 13 and 15 to 18 was used as follows to prepare a carbon dioxide gel for external use. That is, the gel for external use was prepared using 0.3 g of the granular material (A) and 3.0 g of the viscous material (B) of each of the compositions. The resultant gels for external use were each applied to the forearm or the forefoot. All the applied gels caused reactions (skin reddening, cooling sensation and such) which were effected by absorbing the generated carbon dioxide. The carbon dioxide gels for external use prepared from the compositions of Examples 10, 11 were both solidified within 30 minutes, forming hydrogel sheets. On the other hand, the carbon dioxide gels for external use prepared from the compositions of Examples 12, 13, 15 to 18 were not solidified within five minutes from the preparation and could be spread on the skin. These gels were solidified at their surfaces in 15 to 20 minutes and formed hydrogel sheets 30 minutes after the preparation. Thereafter, the individual hydrogel sheets were completely peeled off from the skin and quite favorable results were achieved.

A carbon dioxide gel for external use was prepared using 0.15 g of the granular material (A) and 3.0 g of the viscous material (B) of the composition for use in preparation of carbon dioxide gel for external use of Example 14. The resultant gel for external use was applied to the forearm or the forefoot. The applied gel caused reactions (skin reddening, cooling sensation and such) which were effected by absorbing the generated carbon dioxide. The carbon dioxide gel for external use so applied was solidified within 30 minutes, forming hydrogel sheets.

Each of the compositions for use in preparation of carbon dioxide gel for external use of Examples 19 to 25 was used as follows to prepare a carbon dioxide gel for external use. That is, the gel for external use was prepared using 0.2 g of the granular material (A) and 3.0 g of the viscous material (B) of each of the compositions. The resultant gels for external use were applied to the forearm or the forefoot. All the applied gels caused reactions (skin reddening, cooling sensation and such) which were effected by absorbing the generated carbon dioxide. The carbon dioxide gels for external use prepared from the compositions of Examples 21 to 25 were all solidified within 30 minutes, forming hydrogel sheets. On the other hand, the carbon dioxide gels for external use prepared from the compositions of Examples 19, 20 were not solidified within five minutes from the preparation and could be spread on the skin. These gels were solidified at their surfaces in 15 to 20 minutes and formed hydrogel sheets 30 minutes after the preparation. Thereafter, the individual hydrogel sheets were completely peeled off from the skin and quite favorable results were achieved.

Each of the compositions for use in preparation of carbon dioxide gel for external use of Examples 26 to 36 was used as follows to prepare a carbon dioxide gel for external use. That is, the gel for external use was prepared using 0.3 g of the granular material (A) and 3.0 g of the viscous material (B) of each of the compositions. The resultant gels for external use were applied to the forearm or the forefoot. All the applied gels caused reactions (skin reddening, cooling sensation and such) which were effected by absorbing the generated carbon dioxide. The carbon dioxide gels for external use so applied were not solidified within five minutes from the preparation and could be spread on the skin. These gels were solidified at their surfaces in 15 to 20 minutes and formed hydrogel sheets 30 minutes after the preparation. Thereafter, the individual hydrogel sheets were completely peeled off from the skin and quite favorable results were achieved.

Each of the compositions for use in preparation of carbon dioxide gel for external use of Examples 37 to 40 was used as follows to prepare a carbon dioxide gel for external use. That is, the gel for external use was prepared using 0.3 g of the granular material (A) and 3.0 g of the viscous material (B) of each of the compositions. The resultant gels for external use were applied to the forearm or the forefoot. All the applied gels caused reactions (skin reddening, cooling sensation and such) which were effected by absorbing the generated carbon dioxide. The carbon dioxide gels for external use so applied were all solidified within 30 minutes, forming hydrogel sheets.

Each of the compositions for use in preparation of carbon dioxide gel for external use of Examples 41 to 59 was used as follows to prepare a carbon dioxide gel for external use. That is, the gel for external use was prepared using 0.3 g of the granular material (A) and 3.0 g of the viscous material (B) of each of the compositions. The resultant gels for external use were applied to the forearm or the forefoot. All the applied gels caused reactions (skin reddening, cooling sensation and such) which were effected by absorbing the generated carbon dioxide. The carbon dioxide gels for external use so applied were not solidified within five minutes from the preparation and could be spread on the skin. These gels were solidified at their surfaces in 15 to 20 minutes and formed hydrogel sheets 30 minutes after the preparation. Thereafter, the individual hydrogel sheets were completely peeled off from the skin and quite favorable results were achieved.

Each of the compositions for use in preparation of carbon dioxide gel for external use of Examples 60 to 69 was used as follows to prepare a carbon dioxide gel for external use. That is, the gel for external use was prepared using 0.3 g of the granular material (A) and 3.0 g of the viscous material (B) of each of the compositions. The resultant gels for external use were applied to the forearm or forefoot. All the applied gels caused reactions (skin reddening, cooling sensation and such) which were effected by absorbing the generated carbon dioxide. The carbon dioxide gels for external use prepared from the compositions of Examples 61, 62, 66 to 69 were all solidified within 30 minutes, forming hydrogel sheets. On the other hand, the carbon dioxide gels for external use prepared from the compositions of Examples 60, 63 to 65 were not solidified within five minutes from the preparation and could be spread on the skin. These gels were solidified at their surfaces in 15 to 20 minutes and formed hydrogel sheets 30 minutes after the preparation. Thereafter, the individual hydrogel sheets were completely peeled off from the skin and quite favorable results were achieved.

Each of the compositions for use in preparation of carbon dioxide gel for external use of Examples 70 to 86 was used as follows to prepare a carbon dioxide gel for external use. That is, the gel for external use was prepared using 0.2 g of the granular material (A) and 3.0 g of the viscous material (B) of each of the compositions. The resultant gels for external use were applied to the forearm or the forefoot. All the applied gels caused reactions (skin reddening, cooling sensation and such) which were effected by absorbing the generated carbon dioxide. The carbon dioxide gels for external use prepared from the compositions of Examples 84, 85 were both solidified within 30 minutes, forming hydrogel sheets. On the other hand, the carbon dioxide gels for external use prepared from the compositions of Examples 70 to 83, 86 were not solidified within five minutes from the preparation and could be spread on the skin. These gels were solidified at their surfaces in 15 to 20 minutes and formed hydrogel sheets 30 minutes after the preparation. Thereafter, the individual hydrogel sheets were completely peeled off from the skin and quite favorable results were achieved.

A carbon dioxide gel for external use was prepared using 0.005 g of the granular material (A) and 3.0 g of the viscous material (B) of the composition for use in preparation of carbon dioxide gel for external use of Example 87. The resultant gel for external use was applied to the forearm or the forefoot. The applied gel caused reactions (skin reddening, cooling sensation and such) which were effected by absorbing the generated carbon dioxide. The carbon dioxide gel for external use prepared from the composition of Example 87 was solidified within 30 minutes, forming hydrogel sheets.

A carbon dioxide gel for external use was prepared using 0.05 g of the granular material (A) and 1.0 g of the viscous material (B) of the composition for use in preparation of carbon dioxide gel for external use of Example 88. The resultant gel for external use was applied to the forearm or the forefoot. The applied gel caused reactions (skin reddening, cooling sensation and such) which were effected by absorbing the generated carbon dioxide. The carbon dioxide gel for external use prepared from the composition of Example 88 was solidified within 30 minutes, forming hydrogel sheets.

### Evaluation 6: Wound Treatment (2)

A carbon dioxide gel for external use was prepared using 0.2 g of the granular material (A) and 3.0 g of the viscous material (B) of the composition for use in preparation of carbon dioxide gel for external use of Example 19. The resultant gel was applied to the chapped hand fingers of a 41-year-old woman. Immediately after the preparation, the gel was soft and highly spreadable and hence, was easy to apply. The gel continued to deliver the cooling sensation to the applied area for more than 30 minuets. Thus, the pain associated with the chapped skin was eliminated. After the lapse of 30 minutes from the preparation, the gel was adequately solidified to form a hydrogel sheet, which served to protect the chopped skin for more than six hours before the removal of the sheet.

The results of the above evaluation tests show that the carbon dioxide gels for external use prepared from the compositions for use in preparation of carbon dioxide gel for external use of the present invention provide more rapid and higher aesthetic or medical effects than the kit disclosed in Japanese Patent Application Laid-open No.2000-319187 and the composition disclosed in Publication No.WO02/80941. The gels for external use of the present invention can be applied in uniform and small thickness. When applied, the gels for external use of the present invention do not drop down and are easy to remove after use.

### Industrial Applicability

As described above, the first and second compositions for use in preparation of carbon dioxide gel for external use according to the present invention are adapted to form the carbon dioxide gels for external use in which a large amount of carbon dioxide in the substantial non-bubble form is dissolved. When such a carbon dioxide gel for external use is applied to the skin or mucosa, the higher aesthetic or medical effects can be obtained more rapidly. Therefore, desired aesthetic or medical effects can be achieved by using a smaller amount of a gel for external use in a smaller number of applications. Because of the gel nature, the applied gel for external use does not drop down and is easy to remove after use. Accordingly, the compositions of the present invention can favorably be used as cosmetic materials or medicinal products such as wound covering materials.

## Claims

1. A composition for use in preparation of carbon dioxide gel for external use which is used for preparing a carbon dioxide gel for external use including carbon dioxide dissolved therein in a substantial non-bubble form and which comprises the following granular material (A) and viscous material (B):
(A) a granular material including a weak acid, a dispersant and a calcium ion trapping agent as essential components; and
(B) a viscous material including calcium carbonate, a gelling agent gelated by calcium ions and water as essential components.

2. The composition for according to Claim 1,
wherein the calcium ion trapping agent of the granular material (A) is at least one of disodium ethylenediamine tetraacetate and glycine.

3. The composition according to Claim 1,
- wherein the weak acid of the granular material (A) is at least one of sodium dihydrogenphosphate and potassium dihydrogenphosphate,
- wherein the calcium ion trapping agent of the granular material (A) is at least one of disodium ethylenediamine tetraacetate and glycine, and
- wherein the gelling agent of the viscous material (B), which is gelated by calcium ions, is sodium alginate.

4. The composition according to Claim 1,
wherein the granular material (A) further comprises a binder.

5. The composition according to Claim 1,
wherein the viscous material (B) further comprises an adhesive for increasing affinity to skin or mucosa surface.

6. The composition according to Claim 1,
wherein the viscous material (B) further comprises an alcohol.

7. The composition according to Claim 1,
wherein a mean particle size of the granular material (A) is defined to range from 0.05 mm to 1.0 mm.

8. A composition for use in preparation of carbon dioxide gel for external use which is used for preparing a carbon dioxide gel for external use including carbon dioxide dissolved therein in a substantial non-bubble form and which comprises the following granular material (X) and viscous material (Y):
(X) a granular material including a weak acid and a dispersant as essential components; and
(Y) a viscous material including calcium carbonate, a gelling agent gelated by calcium ions, a calcium ion trapping agent and water as essential components.

9. The composition for according to Claim 8,
wherein the calcium ion trapping agent of the viscous material (Y) is disodium hydrogenphosphate.

10. The composition for according to Claim 8,
- wherein the weak acid of the granular material (X) is at least one of sodium dihydrogenphosphate and potassium dihydrogenphosphate,
- wherein the gelling agent of the viscous material (Y), which is gelated by calcium ions, is sodium alginate, and
- wherein the calcium ion trapping agent of the viscous material (Y) is disodium hydrogenphosphate.

11. The composition according to Claim 8,
wherein the granular material (X) further comprises a binder.

12. The composition according to Claim 8,
wherein the viscous material (Y) further comprises an adhesive for increasing affinity to skin or mucosa surface.

13. The composition for according to Claim 8,
wherein the viscous material (Y) further comprises an alcohol.

14. The composition according to Claim 8,
wherein a mean particle size of the granular material (X) is defined to range from 0.05 mm to 1.0 mm.

15. A carbon dioxide gel for external use which is prepared using the composition according to any one of Claims 1 to 14,
and in which carbon dioxide is dissolved in a substantial non-bubble form.

16. A wound covering material which is prepared using the composition in according to any one of Claims 1 to 14,
and in which carbon dioxide is dissolved in a substantial non-bubble form.

## Patentansprüche

1. Zusammensetzung für die Verwendung bei der Herstellung von einem Kohlendioxidgelpräparat für die äußerliche Anwendung, die zum Herstellen eines Kohlendioxidgels für die äußerliche Anwendung verwendet wird, das Kohlendioxid aufweist, das in einer im wesentlichen blasenfreien Form darin gelöst ist, und die das folgende körnige Material (A) und viskose Material (B) aufweist:
(A) ein körniges Material, das eine schwache Säure, ein Dispersionsmittel und ein Calciumionen einfangendes Mittel als wesentliche Bestandteile aufweist, und
(B) ein viskoses Material, das Calciumcarbonat, ein Geliermittel, das durch Calciumionen geliert, und Wasser als wesentliche Bestandteile aufweist.

2. Zusammensetzung nach Anspruch 1,
wobei das Calciumionen einfangende Mittel des körnigen Materials (A) Dinatriumethylendiamintretaacetat und/oder Glycin ist.

3. Zusammensetzung nach Anspruch 1,
- wobei die schwache Säure des körnigen Materials (A) zumindest eine von Natriumdihydrogenphosphat und Kaliumdihydrogenphosphat ist,
- wobei das Calciumionen einfangende Mittel des körnigen Materials (A) Dinatriumethylendiamintetraacetat und/oder Glycin ist und
- wobei das Geliermittel des viskosen Materials (B), das durch Calciumionen geliert, Natriumalginat ist.

4. Zusammensetzung nach Anspruch 1,
wobei das körnige Material (A) ferner ein Bindemittel aufweist.

5. Zusammensetzung nach Anspruch 1,
wobei das viskose Material (B) ferner ein Klebemittel aufweist, um die Affinität für eine Haut- oder Schleimhautoberfläche zu verbessern.

6. Zusammensetzung nach Anspruch 1,
wobei das viskose Material (B) ferner einen Alkohol aufweist.

7. Zusammensetzung nach Anspruch 1,
wobei die mittlere Partikelgröße des körnigen Materials (A) in einem Bereich von 0,05 mm bis 1,0 mm vorgegeben wird.

8. Zusammensetzung für die Verwendung bei der Herstellung von einem Kohlendioxidgelpräparat für die äußerliche Anwendung, die zum Herstellen eines Kohlendioxidgels für die äußerliche Anwendung verwendet wird, das Kohlendioxid aufweist, das in einer im wesentlichen blasenfreien Form darin gelöst ist, und die das folgende körnige Material (X) und viskose Material (Y) aufweist:
(X) ein körniges Material, das eine schwache Säure und ein Dispersionsmittel als wesentliche Bestandteile aufweist, und
(Y) ein viskoses Material, das Calciumcarbonat, ein Geliermittel, das durch Calciumionen geliert, ein Calciumionen einfangendes Mittel und Wasser als wesentliche Bestandteile aufweist.

9. Zusammensetzung nach Anspruch 8,
wobei das Calciumionen einfangende Mittel des viskosen Materials (Y) Dinatriumhydrogenphosphat ist.

10. Zusammensetzung nach Anspruch 8,
- wobei die schwache Säure des körnigen Materials (X) zumindest eine von Natriumdihydrogenphosphat und Kaliumdihydrogenphosphat ist,
- wobei das Geliermittel des viskosen Materials (Y), das durch Calciumionen geliert, Natriumalginat ist und
- wobei das Calciumionen einfangende Mittel des viskosen Materials (Y) Dinatriumhydrogenphosphat ist.

11. Zusammensetzung nach Anspruch 8,
wobei das körnige Material (X) ferner ein Bindemittel aufweist.

12. Zusammensetzung nach Anspruch 8,
wobei das viskose Material (Y) ferner ein Klebemittel aufweist, um die Affinität für eine Haut- oder Schleimhautoberfläche zu verbessern.

13. Zusammensetzung nach Anspruch 8,
wobei das viskose Material (Y) ferner einen Alkohol aufweist.

14. Zusammensetzung nach Anspruch 8,
wobei die mittlere Partikelgröße des körnigen Materials (X) im Bereich von 0,05 mm bis 1,0 mm vorgegeben wird.

15. Kohlendioxidgel für die äußerliche Anwendung, das unter Verwendung einer Zusammensetzung nach einem der Ansprüche 1 bis 14 hergestellt ist und
in dem Kohlendioxid in einer im wesentlichen blasenfreien Form gelöst ist.

16. Wundabdeckmaterial, das unter Verwendung einer Zusammensetzung nach einem der Ansprüche 1 bis 14 hergestellt ist, und in dem Kohlendioxid in einer im wesentlichen blasenfreien Form gelöst ist.

## Revendications

1. Composition destinée à être utilisée dans la préparation d'un gel de dioxyde de carbone à usage externe qui est utilisée pour préparer un gel de dioxyde de carbone à usage externe incluant du dioxyde de carbone dissous à l'intérieur dans une forme sensiblement sans bulles et qui comprend la matière granulaire (A) et la matière visqueuse (B) suivantes :
(A) une matière granulaire incluant un acide faible, un dispersant et un agent piégeant les ions calcium comme composants essentiels; et
(B) une matière visqueuse incluant du carbonate de calcium, un agent gélifiant gélifié par les ions calcium et de l'eau comme composants essentiels.

2. Composition selon la revendication 1
où l'agent piégeant les ions calcium de la matière granulaire (A) est au moins l'un de l'éthylènediamine tétraacétate de disodium et de la glycine.

3. Composition selon la revendication 1
- où l'acide faible de la matière granulaire (A) est au moins l'un du dihydrogénophosphate de sodium et du dihydrogénophosphate de potassium,
- où l'agent piégeant les ions calcium de la matière granulaire (A) est au moins l'un de l'éthylènediamine tétraacétate de disodium et de la glycine, et
- où l'agent gélifiant de la matière visqueuse (B), qui est gélifié par les ions calcium, est l'alginate de sodium.

4. Composition selon la revendication 1
où la matière granulaire (A) comprend en outre un liant.

5. Composition selon la revendication 1
où la matière visqueuse (B) comprend en outre un adhésif pour augmenter l'affinité avec la peau ou une surface muqueuse.

6. Composition selon la revendication 1
où la matière visqueuse (B) comprend en outre un alcool.

7. Composition selon la revendication 1
où une taille de particule moyenne de la matière granulaire (A) est définie pour aller de 0,05 mm à 1,0 mm.

8. Composition destinée à être utilisée dans la préparation d'un gel de dioxyde de carbone à usage externe qui est utilisée pour préparer un gel de dioxyde de carbone à usage externe incluant du dioxyde de carbone dissous à l'intérieur dans une forme sensiblement sans bulles et qui comprend la matière granulaire (X) et la matière visqueuse (Y) suivantes :
(X) une matière granulaire incluant un acide faible et un dispersant comme composants essentiels; et
(Y) une matière visqueuse incluant du carbonate de calcium, un agent gélifiant gélifié par les ions calcium, un agent piégeant les ions calcium et de l'eau comme composants essentiels.

9. Composition selon la revendication 8
où l'agent piégeant les ions calcium de la matière visqueuse (Y) est l'hydrogénophosphate de disodium.

10. Composition selon la revendication 8
- où l'acide faible de la matière granulaire (X) est au moins l'un du dihydrogénophosphate de sodium et du dihydrogénophosphate de potassium,
- où l'agent gélifiant de la matière visqueuse (Y), qui est gélifié par les ions calcium, est l'alginate de sodium, et
- où l'agent piégeant les ions calcium de la matière visqueuse (Y) est l'hydrogénophosphate de disodium.

11. Composition selon la revendication 8
où la matière granulaire (X) comprend en outre un liant.

12. Composition selon la revendication 8
où la matière visqueuse (Y) comprend en outre un adhésif pour augmenter l'affinité avec la peau ou une surface muqueuse.

13. Composition selon la revendication 8
où la matière visqueuse (Y) comprend en outre un alcool.

14. Composition selon la revendication 8
où une taille de particule moyenne de la matière granulaire (X) est définie pour aller de 0,05 mm à 1,0 mm.

15. Gel de dioxyde de carbone à usage externe qui est préparé au moyen de la composition selon l'une quelconque des revendications 1 à 14 et où le dioxyde de carbone est dissous dans une forme sensiblement sans bulles.

16. Matière de recouvrement des plaies qui est préparée au moyen de la composition selon l'une quelconque des revendications 1 à 14 et où le dioxyde de carbone est dissous dans une forme sensiblement sans bulles.
